# EUROPEAN PATENT SPECIFICATION

(11) **EP 1 187 852 B1**
(45) Date of publication and mention of the grant of the patent: **08.08.2007**
(21) Application number: 00932622.4
(22) Date of filing: 19.05.2000
(51) Int. Cl.: C07K 14/56, C12N 15/63, C12N 15/62, C12N 15/86, C12N 15/863, A61K 38/21

(54) **EXPRESSION AND EXPORT OF INTERFERON-ALPHA PROTEINS AS Fc FUSION PROTEINS**
EXPRESSION UND EXPORT VON INTERFERON-ALPHA PROTEINEN ALS FC FUSIONSPROTEINE
EXPRESSION ET EXPORTATION DE PROTEINES INTERFERON ALPHA EN TANT QUE PROTEINES DE FUSION

(30) Priority: 19.05.1999 US 134895 P
(43) Date of publication of application: 20.03.2002
(73) Proprietor: EMD Lexigen Research Center Corp., Billerica, MA 01821-3936 (US)
(72) Inventor: LO, Kin-Ming, Lexington, MA 02420 (US); SUN, Yaping, Acton, MA 01720 (US); GILLIES, Stephen, D., Carlisle, MA 01741 (US)
(74) Representative: Benz, Jürgen
(86) International application number: PCT/US2000/013827
(87) International publication number: WO 2000/069913

(56) References cited:
- WO-A-97/24137
- US-A- 5 349 053
- US-A- 5 726 044
- LO KIN-MING ET AL: "High level expression and secretion of Fc-X fusion proteins in mammalian cells." PROTEIN ENGINEERING, vol. 11, no. 6, June 1998 (1998-06), pages 495-500, XP002143888 ISSN: 0269-2139

## Description

### Field of the Invention

The invention relates to high level expression and secretion in mammalian cells of Fc fusion proteins, having the biological activity of Interferon-alpha, and the various structural forms and uses thereof, especially their ability to concentrate interferon-alpha in the liver.

### Background of the Invention

The interferon-alpha (IFN-alpha) family of proteins has proven to be useful in treatment of a variety of diseases. For example, interferons alpha 2a and 2b (trade names Roferon and Intron A, respectively) have been used in the treatment of chronic hepatitis B, C and D (life-threatening viral diseases of the liver), condylomata acuminata (genital warts), AIDS-related Kaposi's sarcoma, hairy cell leukemia, malignant melanoma, basal cell carcinoma, multiple myeloma, renal cell carcinoma, herpes I and II, varicella/herpes zoster, and mycosis fungoides. The efficacy of treatment regimes containing interferon-alpha prostate cancer and chronic myelogenous leukemia have also been studied.

The human interferon-alpha family is the largest and most complex family of interferons. Members of the interferon-alpha family have similar amino acid sequences that define them as a group distinct from other interferons; i.e., these proteins typically have at least 35% amino acid identity in a typical protein sequence alignment. The SwissProt database contains numerous human interferon-alpha proteins, including the alternatively named interferon-delta and interferon-omega proteins. These proteins typically are synthesized with a leader sequence of about 23 amino acids, and the mature proteins typically have a molecular weight of about 19 kD. Because these proteins are so similar, when interferon-alpha is obtained from a human or other mammalian source and extensively purified, a mixture of isospecies with varying biological activities often are obtained [Georgiadis et al., U.S. Patent No. 4,732,683]. Similarly, the cDNAs encoding these proteins have sufficiently similar sizes and properties that a single set of procedures can be used to manipulate them for purposes of plasmid construction. Accordingly, it would be useful to have a method for efficiently producing and purifying a single species of interferon-alpha from a mammalian source.

Because of its relatively small size of about 19 kD (Lawn et al. (1981) PROC. NATL. ACAD. Sci. U.S.A. 78: 5435), interferon-alpha can be filtered by the kidney. However, when filtered, interferon-alpha typically is absorbed and metabolized by kidney tubular cells and, therefore, usually is not excreted. According to current clinical practice, formulated interferon-alpha is administered by intramuscular injection, after which its levels in serum decline with a half-life of about 5 hours for interferon-alpha 2a and 2-3 hours for interferon-alpha 2b (PHYSICIANS DESK REFERENCE, 50th edition, 1996: 2145-2147 and 2364-2373).

Furthermore, because of their small size, multiple, frequent injections of interferon-alpha are required (usually daily or 3 times/week), and there can be significant variation in the level of interferon-alpha in the patient. In addition, the injected doses are large, ranging from about 50 micrograms per dose for hairy cell leukemia to 300 micrograms per dose for AIDS-related Kaposi's sarcoma. High levels of circulating interferon-alpha can result in significant side effects, including skin, neurologic, immune and endocrine toxicities. It is thought that the small size of interferon-alpha allows it to pass through the blood-brain barrier and enter the central nervous system, accounting for some of the neurologic side effects. Accordingly, it would be useful to increase the potency and effective serum half-life in patients being treated with interferon-alpha while at the same time minimizing side effects.

Wo 97/24137 discloses a fusion protein, wherein interferon-alpha is fused to the N-terminal of an Fc-portion of an antibody. This molecule is deemed to be suitable for treating hepatitis because it has a longer retention time in the vasculature as compared to the non-fused interferon-alpha. However, as a rule, such a construct mediates ADCC or complement fixation and, therefore, is expected to have reltive low concentration in the liver.

Given the high dosage, low efficacy, short serum half-life, difficulties in purification, and side effects of interferon-alpha, or undesired effects of interferon-alpha to constructs, there is a need in the art for methods of enhancing the production and improving the pharmacological properties of this therapeutic agent.

### Summary of the Invention

The present invention features methods and compositions useful for making and using fusion proteins containing interferon-alpha. In particular, the invention features nucleic acids, for example, DNA or RNA sequences, encoding an immunoglobulin Fc-interferon-alpha fusion protein, and methods for expressing the nucleic acid to produce such fusion proteins. The fusion proteins can facilitate high level expression of biologically active interferon-alpha. The fusion proteins are able to concentrate interferon-alpha in the liver. The fusion protein can be combined with a pharmaceutically acceptable carrier prior to administration to a mammal, for example, a human.

The present invention provides nucleic acid molecules, for example, DNA or RNA molecules, which encode an immunoglobulin Fc region-interferon-alpha fusion protein. The nucleic acid molecule encodes serially in a 5' to 3' direction, a signal sequence, an immunoglobulin Fc region, and at least one target protein, wherein the target protein comprises interferon-alpha.

In a preferred embodiment, the immunoglobulin Fc region comprises an immunoglobulin hinge region and preferably comprises at least one immunoglobulin constant heavy region domain, for example, an immunoglobulin constant heavy 2 (CH2) domain, an immunoglobulin constant heavy 3 (CH3) domain, and depending upon the type of immunoglobulin used to generate the Fc region, optionally an immunoglobulin constant heavy chain 4 (CH4) domain. In a more preferred embodiment, the immunoglobulin Fc region lacks at least an immunoglobulin constant heavy 1 (CH1) domain. Although the immunoglobulin Fc regions may be based on any immunoglobulin class, for example, IgA, IgD, IgE, IgG, and IgM, immunoglobulin Fc regions based on IgG are preferred.

The nucleic acid of the invention can be incorporated in operative association into a replicable expression vector which can then be introduced into a mammalian host cell competent to produce the interferon-alpha-based fusion protein. The resultant interferon-alpha-based fusion protein is produced efficiently and secreted from the mammalian host cell. The secreted interferon-alpha-based fusion protein may be collected from the culture media without lysing the mammalian host cell. The protein product can be assayed for activity and/or purified using common reagents as desired, and/or cleaved from the fusion partner, all using conventional techniques.

The fusion proteins of the present invention demonstrate improved biological properties over native interferon-alpha such as increased solubility, prolonged serum half-life and increased binding to its receptor. These properties may improve significantly the clinical efficacy of interferon-alpha. In a preferred embodiment, the fusion protein comprises, in an N- to C- terminal direction, an immunoglobulin Fc region and interferon-alpha, with other moieties, for example, a proteolytic cleavage site, optionally interposed between the immunoglobulin Fc region and the interferon-alpha. The resulting fusion protein preferably is synthesized in a cell that glycosylates the Fc region at normal glycosylation sites, i. e., which usually exist in template antibodies.

In another embodiment, the fusion protein may comprise a second target protein, for example, mature, full length interferon-alpha or a bioactive fragment thereof. In this type of construct the first and second target proteins can be the same or different proteins. The first and second target proteins may be linked together, either directly or by means of a polypeptide linker. Alternatively, both target proteins may be linked either directly or via a polypeptide linker, to the immunoglobulin Fc region. In the latter case, the first target protein can be connected to an N-terminal end of the immunoglobulin Fc region and the second target protein can be connected to a C-terminal end of the immunoglobulin Fc region.

In another aspect, the invention provides methods of producing a fusion protein comprising an immunoglobulin Fc region and the target protein. The method comprises the steps of (a) providing a mammalian cell containing a DNA molecule encoding such a fusion protein, either with or without a signal sequence, and (b) culturing the mammalian cell to produce the fusion protein. The resulting fusion protein can then be harvested, refolded, if necessary, and purified using conventional purification techniques well known and used in the art.

In yet another aspect, the invention provides methods for treating conditions alleviated by interferon-alpha or active variants thereof by administering to a mammal an effective amount of interferon-alpha produced by a fusion construct of the invention.

In a preferred embodiment, the constructs of the invention can be used in the treatment of a liver disorder, wherein the interferon-alpha by virtue of the immunoglobulin Fc region becomes localized within the liver. The constructs of the invention may be particularly useful in the treatment of liver disorders which include, but are not limited to, viral diseases such as hepatitis B, hepatitis C or hepatitis D, liver cancer as well as other types of cancer involving metastases located in the liver.

The foregoing and other objects, features and advantages of the invention will be apparent from the description, drawings, and claims that follow.

### Brief Description of the Drawings

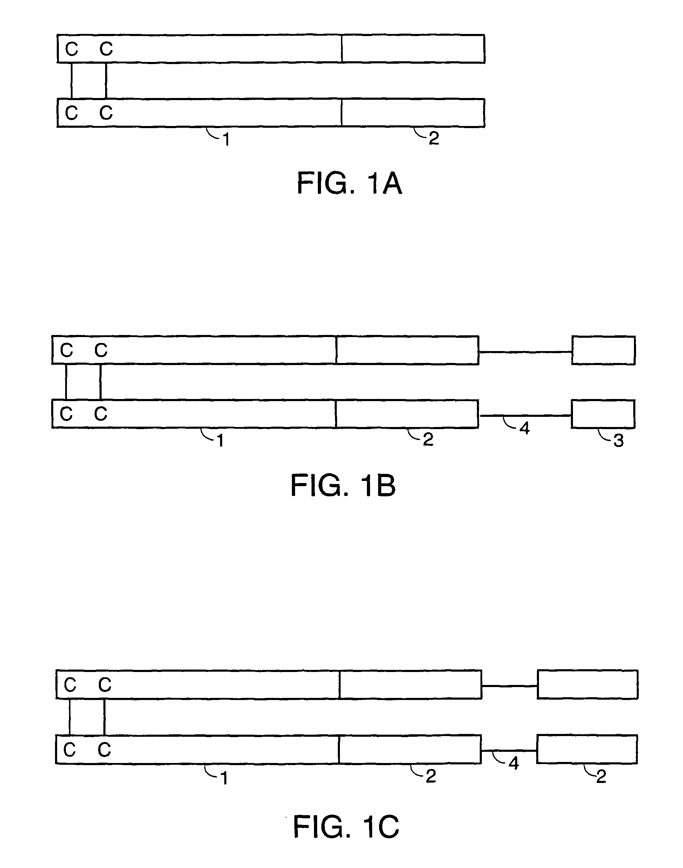

Figures 1A-1C are schematic illustrations of non-limiting examples of fusion proteins constructed in accordance with the invention.

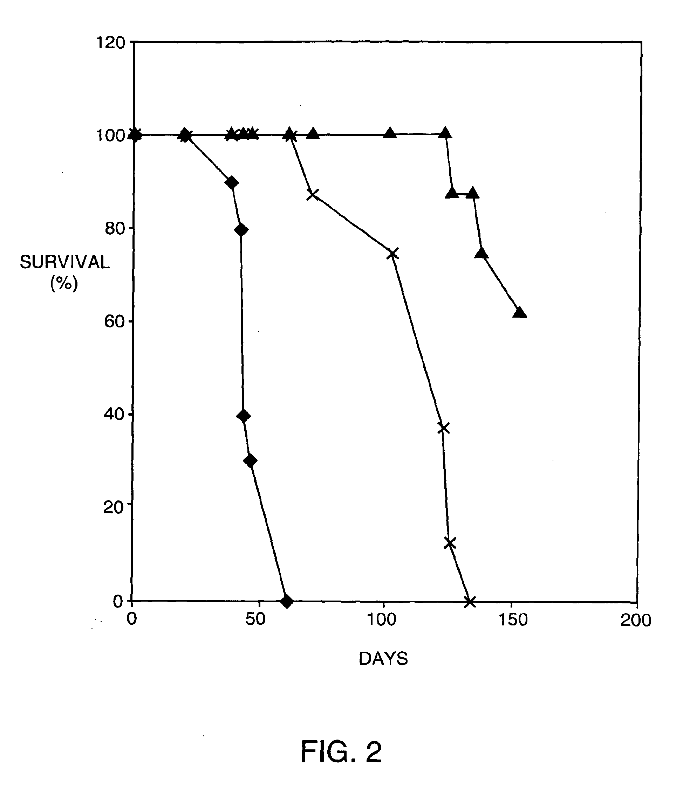

Figure 2 is a graph showing the survival curves for groups of SCID mice injected with suspensions of Daudi cells and then treated with huFc-huIFN-alpha. On day 0, mice were injected with Daudi cells. On days 3-8, groups of eight mice were injected with PBS (diamonds), 30 µg of huFc-huIFN-alpha (crosses), or with 60 µg of huFc-huIFN-alpha (triangles).

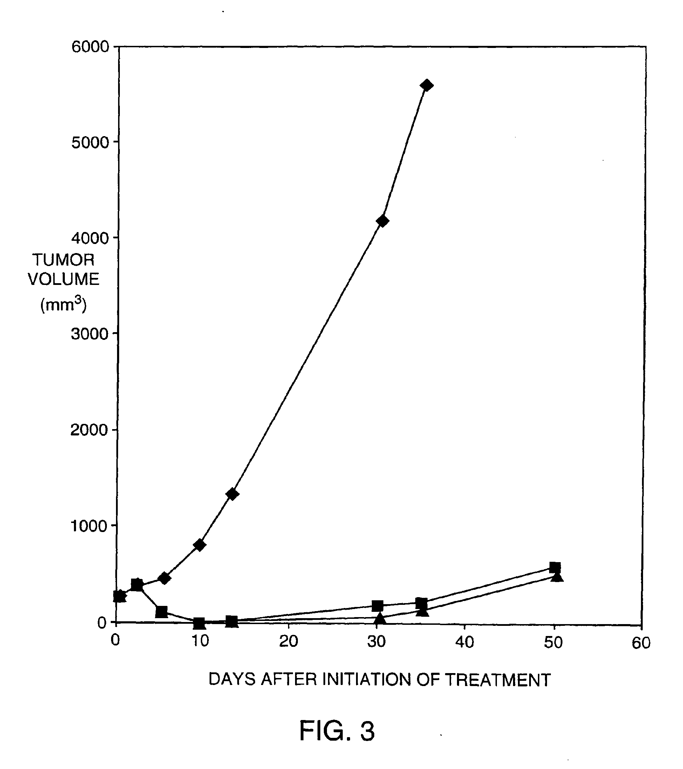

Figure 3 is a graph showing the growth rates of subcutaneous tumors of Daudi cells in SCID mice treated with huFc-huIFN-alpha. About four weeks prior to treatment, mice were subcutaneously injected with Daudi cells. When the injected Daudi cells had grown to form tumors of 200-400 mm³, mice were sorted in groups of eight and treated for six days with an injection of PBS (diamonds), 30 µg of huFc-huIFN-alpha in PBS (squares), or 60 µg ofhuFc-huIFN-alpha in PBS (triangles).

### Detailed Description of the Invention

Many conditions may be alleviated by the administration of interferon-alpha. For example, as discussed previously, interferons alpha 2a and 2b (trade names Roferon and Intron A, respectively) are useful in the treatment of chronic hepatitis B, C and D, condylomata acuminata (genital warts), AIDS-related Kaposi's sarcoma, hairy cell leukemia, malignant melanoma, basal cell carcinoma, multiple myeloma, renal cell carcinoma, herpes I and II, varicella/herpes zoster, and mycosis fungoides. Furthermore, studies have been performed to evaluate the efficacy of interferon-alpha in the treatment of prostate cancer and chronic myelogenous leukemia.

For the treatment of hepatitis, for example, it can be particularly useful to have a form of interferon-alpha which is concentrated in the liver. In this way, the concentration of interferon-alpha in other tissues can be minimized, thereby reducing side effects. Liver tissue is the primary site for removal of soluble immune complexes, and Fc receptors are abundant on liver macrophages (Kupffer cells) (Benacerraf, B. et al. (1959) J. IMMUNOL. 82: 131; Paul, W. E. (1993) FUNDAMENTALS OF IMMUNOLOGY, 3rd ed. ch. 5:113-116). Accordingly, by fusing interferon-alpha to an immunoglobulin Fc region, the interferon-alpha molecule can be targeted preferably to liver tissue relative to the same interferon-alpha molecule lacking the immunoglobulin Fc region. The IgG type of antibody that has the highest affinity for the Fc receptors are IgG1. However, in contrast, IgG4, for example, has an approximately 10-fold lower affinity for the Fc gamma receptor I (Anderson and Abraham (1980) J. IMMUNOL. 125: 2735; Woof *et al.* (1986) MOL. IMMUNOL. 23: 319). Fc-gamma 1 from IgG1, when placed at the C-terminus of a ligand, can mediate antibody-dependent cell-mediated cytotoxicity (ADCC) against cells that express a receptor for that ligand. In addition, Fc-gamma 1, when present on the C-terminus of a ligand, can mediate C1q binding and complement fixation directed against cells expressing a receptor for that ligand.

In contrast to IgG1, IgG4 does not effectively fix complement. This has led to the proposal that an N-terminal interferon-alpha could be fused to a C-terminal Fc region from IgG4 (Chang, T. W. et al., U. S. Patent No. 5,723,125). However, when the Fc region of IgG4 is separated from the Fab region, the Fc of IgG4 fixes complement as well as the Fc region of IgG1 (Isenman, D. E. et al. (1975) J. IMMUNOL. 114: 1726). Based on this result and the fact that the Fc sequences of IgG 1 and IgG4 are quite similar, without wishing to be bound by theory, it is contemplated that the Fab region of IgG4 sterically blocks C 1 q binding and complement fixation because the hinge region connecting the IgG4 Fab and Fc regions is shorter than the hinge of IgG1. If the large, bulky Fab region of IgG4 is replaced by a small molecule, such as interferon-alpha, and the interferon-alpha and Fc region are connected by a flexible linker, it is contemplated that such an interferon-alpha-Fc-gamma 4 fusion would fix complement when bound to cells bearing interferon-alpha receptors.

The cytotoxic effect due to the fusion of an N-terminal cytokine and a C-terminal Fc region is well known. For example, fusion of the cytokine interleukin-2 (IL-2) to an Fc region creates a molecule that is able to fix complement and cause lysis of cells bearing the IL-2 receptor (Landolfi, N. F., U. S. Patent No. 5,349,053).

Fusions in which an Fc region is placed at the N-terminus of a ligand (termed 'immunofusins' or'Fc-X' fusions, where X is a ligand such as Interferon-alpha) have a number of distinctive, advantageous biological properties (Lo et al., U. S. Patent Nos. 5,726,044 and 5,541,087; Lo et al. (1998) PROTEIN ENGINEERING 11: 495). In particular, such fusion proteins can still bind to the relevant Fc receptors on cell surfaces. However, when the ligand binds to its receptor on a cell surface, the orientation of the Fc region is altered and the sequences that mediate ADCC and complement fixation appear to be occluded. As a result, the Fc region in an Fc-X molecule does not mediate ADCC or complement fixation effectively. Thus, Fc-X fusions are expected to have the virtues of increased serum half-life and relative concentration in the liver, with little deleterious effects from ADCC and complement fixation.

One feature of the Fc-X constructs of the invention is to concentrate the target protein, in this case interferon-alpha, in the liver. The Fc region from the gamma1 and gamma3 chains show the highest affinity for the Fc receptor, with the gamma4 chain showing a reduced affinity and the gamma2 chain showing extremely low affinity to the Fc receptor. Accordingly, Fc regions derived from gamma1 or gamma3 chains preferably are used in the Fc-X constructs of the invention because they have the highest affinities for Fc receptors and thus can target the interferon-alpha preferentially to liver tissues. This is in contrast to an X-Fc protein, for example, an interferon-alpha-Fc fusion protein where the potential advantage of concentration in the liver must be balanced by the fact that this fusion protein can mediate effector functions, namely complement fixation and ADCC, directed against cells bearing receptors for interferon-alpha.

The invention thus provides nucleic acid sequences encoding and amino acid sequences defining fusion proteins comprising an immunoglobulin Fc region and at least one target protein, referred to herein as interferon-alpha. Three exemplary embodiments of protein constructs embodying the invention are illustrated in the drawing as Figures 1A-1C. Because dimeric constructs are preferred, all are illustrated as dimers cross-linked by a pair of disulfide bonds between cysteines in adjacent subunits. In the drawings, the disulfide bonds are depicted as linking together the two immunoglobulin heavy chain Fc regions via an immunoglobulin hinge region within each heavy chain, and thus are characteristic of native forms of these molecules. While constructs including the hinge region of Fc are preferred and have been shown promise as therapeutic agents, the invention contemplates that the crosslinking at other positions may be chosen as desired. Furthermore, under some circumstances, dimers or multimers useful in the practice of the invention may be produced by non-covalent association, for example, by hydrophobic interaction. Because homodimeric constructs are important embodiments of the invention, the drawings illustrate such constructs. It should be appreciated, however, that heterodimeric structures also are useful in the practice of the invention.

Figure 1 A illustrates a dimeric construct produced in accordance with the principles set forth herein (see, for example, Example 1). Each monomer of the homodimer comprises an immunoglobulin Fc region 1 including a hinge region, a CH2 domain and a CH3 domain. Attached directly, i.e., via a polypeptide bond, to the C terminus of the Fc region is interferon-alpha 2. It should be understood that the Fc region may be attached to a target protein via a polypeptide linker (not shown).

Figures 1B and 1C depict protein constructs of the invention which include as a target protein plural interferon-alpha proteins arranged in tandem and connected by a linker. In Figure 1B, the target protein comprises full length interferon-alpha **2,** a polypeptide linker made of glycine and serine residues **4,** and an active variant of interferon-alpha **3.** Figure 1C differs from the construct of Figure 1B in that the most C-terminal protein domain comprises a second, full length copy of interferon-alpha **2.** Although Figures 1A-1C represent Fc-X constructs, where X is the target protein, it is contemplated that useful proteins of the invention may also be depicted by the formula X-Fc-X, wherein the X's may represent the same or different target proteins.

As used herein, the term "polypeptide linker" is understood to mean a polypeptide sequence that can link together two proteins that in nature are not naturally linked together. The polypeptide linker preferably comprises a plurality of amino acids such as alanine, glycine and serine or combinations of such amino acids. Preferably, the polypeptide linker comprises a series of glycine and serine peptides about 10-15 residues in length. See, for example, U.S. Patent No. 5,258,698. It is contemplated, however, that the optimal linker length and amino acid composition may be determined by routine experimentation.

As used herein, the term "multivalent" refers to a recombinant molecule that incorporates two or more biologically active segments. The protein fragments forming the multivalent molecule optionally may be linked through a polypeptide linker which attaches the constituent parts and permits each to function independently.

As used herein, the term "bivalent" refers to a multivalent recombinant molecule having the configuration Fc-X or X-Fc, where X is a target molecule. The immunoglobulin Fc regions can associate, for example, via interchain disulfide bonds, to produce the type of constructs shown in Figs. 1 A. If the fusion construct of the invention has the configuration Fc-X-X, the resulting Fc molecule is shown in Fig. 1C. The two target proteins may be linked through a peptide linker. Constructs of the type shown in Fig. 1A can increase the apparent binding affinity between the target molecule and its receptor.

As used herein, the term "multimeric" refers to the stable association of two or more polypeptide chains either covalently, for example, by means of a covalent interaction, for example, a disulfide bond, or non-covalently, for example, by hydrophobic interaction. The term multimer is intended to encompass both homomultimers, wherein the subunits are the same, as well as, heteromultimers, wherein the subunits are different.

As used herein, the term "dimeric" refers to a specific multimeric molecule where two polypeptide chains are stably associated through covalent or non-covalent interactions. Such constructs are shown schematically in Fig. 1A. It should be understood that the immunoglobulin Fc region including at least a portion of the hinge region, a CH2 domain and a CH3 domain, typically forms a dimer. Many protein ligands are known to bind to their receptors as a dimer. If a protein ligand X dimerizes naturally, the X moiety in an Fc-X molecule will dimerize to a much greater extent, since the dimerization process is concentration dependent. The physical proximity of the two X moieties connected by Fc would make the dimerization an intramolecular process, greatly shifting the equilibrium in favor of the dimer and enhancing its binding to the receptor.

As used herein, the term "interferon-alpha" is understood to mean not only full length mature interferon-alpha, for example, human interferon-alpha 1 (SEQ ID NO: 8), human interferon-alpha 2 (SEQ ID NO: 9), human interferon-alpha 4 (SEQ ID NO: 10), human interferon-alpha 5 (SEQ ID NO: 11), human interferon-alpha 6 (SEQ ID NO: 12), human interferon-alpha 7 (SEQ ID NO: 13), human interferon-alpha 8 (SEQ ID NO: 14), human interferon-alpha 10 (SEQ ID NO: 15), human interferon-alpha 14 (SEQ ID NO: 16), human interferon-alpha 16 (SEQ ID NO: 17), human interferon-alpha 17 (SEQ ID NO: 18), human interferon-alpha 21 (SEQ ID NO: 19), interferon delta-1 (SEQ ID NO: 20), II-1 (interferon omega-1) (SEQ ID NO: 21); and mouse interferon-alpha 1 (SEQ ID NO: 22), mouse interferon-alpha 2 (SEQ ID NO: 23), mouse interferon-alpha 4 (SEQ ID NO: 24), mouse interferon-alpha 5 (SEQ ID NO: 25), mouse interferon-alpha 6 (SEQ ID NO: 26), mouse interferon-alpha 7 (SEQ ID NO: 27), mouse interferon-alpha 8 (SEQ ID NO 28), and mouse interferon-alpha 9 (SEQ ID NO: 29), but also variants and bioactive fragments thereof. Known sequences of interferon-alpha may be found in GenBank.

The term bioactive fragment refers to any interferon-alpha protein fragment that has at least 50%, more preferably at least 70%, and most preferably at least 90% of the biological activity of the template human interferon-alpha protein of SEQ ID NO: 2, as determined using the cell proliferation inhibition assay of Example 4. The term variants includes species and allelic variants, as well as other naturally occurring or non-naturally occurring variants, for example, generated by genetic engineering protocols, that are at least 70% similar or 60% identical, more preferably at least 75% similar or 65% identical, and most preferably at least 80% similar or 70% identical to the mature human interferon-alpha protein disclosed in SEQ ID NO.: 2.

To determine whether a candidate polypeptide has the requisite percentage similarity or identity to a reference polypeptide, the candidate amino acid sequence and the reference amino acid sequence are first aligned using the dynamic programming algorithm described in Smith and Waterman (1981) J. MOL. BIOL. 147:195-197, in combination with the BLOSUM62 substitution matrix described in Figure 2 of Henikoff and Henikoff (1992), "Amino acid substitution matrices from protein blocks", PROC. NATL. ACAD. SCI. USA 89:10915-10919. For the present invention, an appropriate value for the gap insertion penalty is -12, and an appropriate value for the gap extension penalty is -4. Computer programs performing alignments using the algorithm of Smith-Waterman and the BLOSUM62 matrix, such as the GCG program suite (Oxford Molecular Group, Oxford, England), are commercially available and widely used by those skilled in the art.

Once the alignment between the candidate and reference sequence is made, a percent similarity score may be calculated. The individual amino acids of each sequence are compared sequentially according to their similarity to each other. If the value in the BLOSUM62 matrix corresponding to the two aligned amino acids is zero or a negative number, the pair-wise similarity score is zero; otherwise the pair-wise similarity score is 1.0. The raw similarity score is the sum of the pair-wise similarity scores of the aligned amino acids. The raw score then is normalized by dividing it by the number of amino acids in the smaller of the candidate or reference sequences. The normalized raw score is the percent similarity. Alternatively, to calculate a percent identity, the aligned amino acids of each sequence again are compared sequentially. If the amino acids are non-identical, the pair-wise identity score is zero; otherwise the pair-wise identity score is 1.0. The raw identity score is the sum of the identical aligned amino acids. The raw score is then normalized by dividing it by the number of amino acids in the smaller of the candidate or reference sequences. The normalized raw score is the percent identity. Insertions and deletions are ignored for the purposes of calculating percent similarity and identity. Accordingly, gap penalties are not used in this calculation, although they are used in the initial alignment.

Variants may also include other interferon-alpha mutant proteins having interferon-alpha-like activity. Species and allelic variants, include, but are not limited to human and mouse interferon-alpha sequences. Human interferon-alpha variants are shown in SEQ ID NOS: 8-21, and mouse interferon-alpha variants are shown in SEQ ID NOS: 22-29.

Furthermore, the interferon-alpha sequence may comprise a portion or all of the consensus sequence set forth in SEQ ID NO: 7, wherein the interferon-alpha has at least 50%, more preferably at least 70%, and most preferably at least 90% of the biological activity of the mature human interferon-alpha of SEQ ID NO: 2, as determined using the cell proliferation inhibition assay of Example 4.

These proteins have very similar purification properties and other biological properties. In particular, the DNA manipulation, fusion protein expression, and fusion protein purification properties of Fc-Interferon-alpha proteins are extremely similar. For example, human interferon-alpha 2a and human interferon-alpha 2b differ by one amino acid only, whereas the interferon-alpha 2a has a lysine residue at the same position that interferon-alpha 2b has an arginine residue. Human interferon-alpha 2a and human interferon-alpha 2b have extremely similar properties and are interchangeable for all known purposes.

The three-dimensional structure of interferon-alpha has been solved by X-ray crystallography (Ramaswamy et al. (1986) STRUCTURE 4: 1453). The sequences of interferon-alpha proteins are so similar that the determined structure is regarded as a structure for the entire family of proteins. The three-dimensional structure of interferon-alpha, like that of interferon-beta, is a dimer with a zinc ion at the dimer interface. However, in solution, interferon-alpha behaves as a monomer. It has been proposed, by analogy with the cytokine IL-6 and other protein ligands, that interferon-alpha may dimerize upon receptor binding (Radhakrishnan, R. et al. (1996) STRUCTURE 4: 1453; Karpusas, M. et al. (1997) PROC. NAT. ACAD. SCI. USA 94: 11813).

Dimerization of a ligand can increase the apparent binding affinity between the ligand and its receptor. For instance, if one interferon-alpha moiety of an Fc-Interferon-alpha fusion protein can bind to a receptor on a cell with a certain affinity, the second interferon-alpha moiety of the same Fc-Interferon-alpha fusion protein may bind to a second receptor on the same cell with a much higher avidity (apparent affinity). This may occur because of the physical proximity of the second interferon-alpha moiety to the receptor after the first interferon-alpha moiety already is bound. In the case of an antibody binding to an antigen, the apparent affinity may be increased by at least ten thousand-fold, i.e., 10⁴. Each protein subunit, i.e., "X," has its own independent function so that in a multivalent molecule, the functions of the protein subunits may be additive or synergistic. Thus, fusion of the normally dimeric Fc molecule to interferon-alpha may increase the activity of interferon-alpha. Accordingly, constructs of the type shown in Figure 1A may increase the apparent binding affinity between interferon-alpha and its receptor.

The target proteins disclosed herein are expressed as fusion proteins with an Fc region of an immunoglobulin. As is known, each immunoglobulin heavy chain constant region comprises four or five domains. The domains are named sequentially as follows: CH1-hinge-CH2-CH3(-CH4). The DNA sequences of the heavy chain domains have cross-homology among the immunoglobulin classes, e.g., the CH2 domain of IgG is homologous to the CH2 domain of IgA and IgD, and to the CH3 domain of IgM and IgE.

As used herein, the term, "immunoglobulin Fc region" is understood to mean the carboxyl-terminal portion of an immunoglobulin chain constant region, preferably an immunoglobulin heavy chain constant region, or a portion thereof. For example, an immunoglobulin Fc region may comprise 1) a CH1 domain, a CH2 domain, and a CH3 domain, 2) a CH1 domain and a CH2 domain, 3) a CH1 domain and a CH3 domain, 4) a CH2 domain and a CH3 domain, or 5) a combination of two or more domains and an immunoglobulin hinge region. In a preferred embodiment the immunoglobulin Fc region comprises at least an immunoglobulin hinge region a CH2 domain and a CH3 domain, and preferably lacks the CH1 domain.

The currently preferred class of immunoglobulin from which the heavy chain constant region is derived is IgG (Igγ) (γ subclasses 1, 2, 3, or 4). The nucleotide and amino acid sequences of human Fc γ-1 are set forth in SEQ ID NOS: 3 and 4. Other classes of immunoglobulin, IgA (Igα), IgD (Igδ), IgE (Igε) and IgM (Igµ), may be used. The choice of appropriate immunoglobulin heavy chain constant regions is discussed in detail in U.S. Patent Nos. 5,541,087, and 5,726,044. The choice of particular immunoglobulin heavy chain constant region sequences from certain immunoglobulin classes and subclasses to achieve a particular result is considered to be within the level of skill in the art. The portion of the DNA construct encoding the immunoglobulin Fc region preferably comprises at least a portion of a hinge domain, and preferably at least a portion of a CH₃ domain of Fcγ or the homologous domains in any of IgA, IgD, IgE, or IgM.

Depending on the application, constant region genes from species other than human, for example, mouse or rat may be used. The immunoglobulin Fc region used as a fusion partner in the DNA construct generally may be from any mammalian species. Where it is undesirable to elicit an immune response in the host cell or animal against the Fc region, the Fc region may be derived from the same species as the host cell or animal. For example, a human immunoglobulin Fc region can be used when the host animal or cell is human; likewise, a murine immunoglobulin Fc region can be used where the host animal or cell will be a mouse.

Nucleic acid sequences encoding, and amino acid sequences defining a human immunoglobulin Fc region useful in the practice of the invention are set forth in SEQ ID NOS: 3 and 4. However, it is contemplated that other immunoglobulin Fc region sequences useful in the practice of the invention may be found, for example, by those encoded by nucleotide sequences disclosed in the Genbank and/or EMBL databases, for example, AF045536.1 (*Macaca fuscicularis*), AF045537.1 (*Macaca mulatta*), AB016710 (*Felix catus*), K00752 *(Oryctolagus cuniculus),* U03780 (*Sus scrofa*), Z48947 (*Camelus dromedarius*), X62916 *(Bos taurus),* L07789 (*Mustela vison*), X69797 (*Ovis aries*)*,* U17166 (*Cricetulus migratorius*), X07189 (*Rattus rattus*), AF57619.1 (*Trichosurus vulpecula*), or AF035195 (*Monodelphis domestica*), the disclosures of which are incorporated by reference herein.

Furthermore, it is contemplated that substitution or deletion of amino acids within the immunoglobulin heavy chain constant regions may be useful in the practice of the invention. One example may include introducing amino acid substitutions in the upper CH2 region to create a Fc variant with reduced affinity for Fc receptors (Cole et al. (1997) J. IMMUNOL. 159:3613). One of ordinary skill in the art can prepare such constructs using well known molecular biology techniques.

The use of human Fcγl as the Fc region sequence has several advantages. For example, if the Fc fusion protein is to be used as a biopharmaceutical, the Fcγl domain may confer effector function activities to the fusion protein. The effector function activities include the biological activities such as placental transfer and increased serum half-life. The immunoglobulin Fc region also provides for detection by anti-Fc ELISA and purification through binding to *Staphylococcus aureus* protein A ("Protein A"). In certain applications, however, it may be desirable to delete specific effector functions from the immunoglobulin Fc region, such as Fc receptor binding and/or complement fixation.

It is understood that the present invention exploits conventional recombinant DNA methodologies for generating the Fc fusion proteins useful in the practice of the invention. The Fc fusion constructs preferably are generated at the DNA level, and the resulting DNAs integrated into expression vectors, and expressed to produce the fusion proteins of the invention. As used herein, the term "vector" is understood to mean any nucleic acid comprising a nucleotide sequence competent to be incorporated into a host cell and to be recombined with and integrated into the host cell genome, or to replicate autonomously as an episome. Such vectors include linear nucleic acids, plasmids, phagemids, cosmids, RNA vectors, viral vectors and the like. Non-limiting examples of a viral vector include a retrovirus, an adenovirus and an adeno-associated virus. As used herein, the term "gene expression" or "expression" of a target protein, is understood to mean the transcription of a DNA sequence, translation of the mRNA transcript, and secretion of an Fc fusion protein product.

A useful expression vector is pdCs (Lo et al. (1988) PROTEIN ENGINEERING 11:495, in which the transcription of the Fc-X gene utilizes the enhancer/promoter of the human cytomegalovirus and the SV40 polyadenylation signal. The enhancer and promoter sequence of the human cytomegalovirus used was derived from nucleotides -601 to +7 of the sequence provided in Boshart et al. (1985) CELL 41:521. The vector also contains the mutant dihydrofolate reductase gene as a selection marker (Simonsen and Levinson (1983) PROC. NAT. ACAD. Sci. USA 80:2495).

An appropriate host cell can be transformed or transfected with the DNA sequence of the invention, and utilized for the expression and/or secretion of the target protein. Currently preferred host cells for use in the invention include immortal hybridoma cells, NS/O myeloma cells, 293 cells, Chinese hamster ovary cells, HELA cells, and COS cells.

One expression system that has been used to produce high level expression of fusion proteins in mammalian cells is a DNA construct encoding, in the 5' to 3' direction, a secretion cassette, including a signal sequence and an immunoglobulin Fc region, and a target protein. Several target proteins have been expressed successfully in such a system and include, for example, IL2, CD26, Tat, Rev, OSF-2, βIG-H3, IgE Receptor, PSMA, and gp120. These expression constructs are disclosed in U.S. Patent Nos. 5,541,087 and 5,726,044 to Lo *et al.*

As used herein, the term "signal sequence" is understood to mean a segment which directs the secretion of the interferon-alpha fusion protein and thereafter is cleaved following translation in the host cell. The signal sequence of the invention is a polynucleotide which encodes an amino acid sequence which initiates transport of a protein across the membrane of the endoplasmic reticulum. Signal sequences which are useful in the invention include antibody light chain signal sequences, e.g., antibody 14.18 (Gillies et. al. (1989) J. IMMUNOL. METH. 125:191), antibody heavy chain signal sequences, e.g., the MOPC141 antibody heavy chain signal sequence (Sakano et al. (1980) NATURE 286:5774), and any other signal sequences which are known in the art (see, e.g., Watson (1984) NUCLEIC ACIDS RESEARCH 12:5145).

Signal sequences have been well characterized in the art and are known typically to contain 16 to 30 amino acid residues, and may contain greater or fewer amino acid residues. A typical signal peptide consists of three regions: a basic N-terminal region, a central hydrophobic region, and a more polar C-terminal region. The central hydrophobic region contains 4 to 12 hydrophobic residues that anchor the signal peptide across the membrane lipid bilayer during transport of the nascent polypeptide. Following initiation, the signal peptide is usually cleaved within the lumen of the endoplasmic reticulum by cellular enzymes known as signal peptidases. Potential cleavage sites of the signal peptide generally follow the "(-3, -1) rule". Thus a typical signal peptide has small, neutral amino acid residues in positions -1 and -3 and lacks proline residues in this region. The signal peptidase will cleave such a signal peptide between the -1 and +1 amino acids. Thus, the signal sequence may be cleaved from the amino-terminus of the fusion protein during secretion. This results in the secretion of an Fc fusion protein consisting of the immunoglobulin Fc region and the target protein. A detailed discussion of signal peptide sequences is provided by von Heijne (1986) NUCLEIC ACIDS RES. 14:4683.

As would be apparent to one of skill in the art, the suitability of a particular signal sequence for use in the secretion cassette may require some routine experimentation. Such experimentation will include determining the ability of the signal sequence to direct the secretion of an Fc fusion protein and also a determination of the optimal configuration, genomic or cDNA, of the sequence to be used in order to achieve efficient secretion of Fc fusion proteins. Additionally, one skilled in the art is capable of creating a synthetic signal peptide following the rules presented by von Heijne, referenced above, and testing for the efficacy of such a synthetic signal sequence by routine experimentation. A signal sequence can also be referred to as a "signal peptide," "leader sequence," or "leader peptides."

The fusion of the signal sequence and the immunoglobulin Fc region is sometimes referred to herein as secretion cassette. An exemplary secretion cassette useful in the practice of the invention is a polynucleotide encoding, in a 5' to 3' direction, a signal sequence of an immunoglobulin light chain gene and an Fcγl region of the human immunoglobulin γ1 gene. The Fcγl region of the immunoglobulin Fcγl gene preferably includes at least a portion of the immunoglobulin hinge domain and at least the CH3 domain, or more preferably at least a portion of the hinge domain, the CH2 domain and the CH3 domain. As used herein, the "portion" of the immunoglobulin hinge region is understood to mean a portion of the immunoglobulin hinge that contains at least one, preferably two cysteine residues capable of forming interchain disulfide bonds. The DNA encoding the secretion cassette can be in its genomic configuration or its cDNA configuration. Under certain circumstances, it may be advantageous to produce the Fc region from human immunoglobulin Fcγ2 heavy chain sequences. Although Fc fusions based on human immunoglobulin γ1 and γ2 sequences behave similarly in mice, the Fc fusions based on the γ2 sequences can display superior pharmacokinetics in humans.

Further, substitution or deletion of constructs of these constant regions, in which one or more amino acid residues of the constant region domains are substituted or deleted also would be useful. One example would be to introduce amino acid substitutions in the upper CH2 region to create an Fc variant with reduced affinity for Fc receptors (Cole et al. (1997) J. IMMUNOL. 159: 3613). One of ordinary skill in the art can prepare such constructs using well known molecular biology techniques.

In the Examples disclosed herein, high levels of Fc-Interferon-alpha were produced. The initial clones produced about 50 µg/mL of Fc-Interferon-alpha, which could be purified readily to homogeneity by Protein A affinity chromatography. Expression levels often can be increased several fold by subcloning. As stated above, it is found that when interferon-alpha is expressed as Fc fusion molecules, high levels of expression are obtained, presumably because the Fc portion acts as a carrier, helping the polypeptide at the C-terminus to fold correctly and to be secreted efficiently. Moreover, the Fc region is glycosylated and highly charged at physiological pH, thus the Fc region can help to solubilize hydrophobic proteins.

In addition to the high levels of expression, interferon-alpha fusion proteins exhibited longer serum half-lives compared to interferon-alpha alone, due in part to their larger molecular sizes. For example, Fc-Interferon-alpha has a circulating half-life of 19.3 hours in mouse (see Example 6), as compared to 2-5 hours for interferon-alpha (PHYSICIANS DESK REFERENCE, 50th edition, 1996:2156-2147 and 2364-2373). Interferon-alpha, having a molecular weight of about 19 kD, is small enough to be cleared efficiently by renal filtration. In contrast, Fc-Interferon-alpha has a molecular weight of about 100 kD since there are two interferon-alpha moieties attached to each Fc molecule (i.e., two interferon-alphas since Fc is in its dimeric form). Such a dimeric structure may exhibit a higher binding affinity to the interferon-alpha receptor. Since the interferon-alpha activity is receptor-mediated, the bivalent interferon-alpha fusion proteins will be potentially more efficacious than interferon-alpha itself.

Additionally, many protein ligands are known to bind to their receptors as a dimer. Since interferon-alpha belongs to a class of protein ligands with weak dimerization constants, the physical constraint imposed by the Fc on interferon-alpha would make the dimerization an intramolecular process, thus, shifting the equilibrium in favor of the dimer and enhancing its binding to the receptors. Cysteine residues also can be introduced by standard recombinant DNA technology to the monomer at appropriate places to stabilize the dimer through covalent disulfide bond formation.

The fusion proteins of the invention provide several important clinical benefits. As demonstrated in the tests of biological activity in the Daudi cell and cytopathic effect assays (Example 4), the biological activity of Fc-Interferon-alpha is significantly higher than that of interferon-alpha.

An important aspect of the invention is that the sequences and properties of various interferon-alpha proteins and encoding DNAs are quite similar. In the context of Fc-X fusions, the properties of interferon-alpha proteins and encoding DNAs are essentially identical, so that a common set of techniques can be used to generate any Fc-Interferon-alpha DNA fusion, to express the fusion, to purify the fusion protein, and to administer the fusion protein for therapeutic purposes.

The present invention also provides methods for the production of interferon-alpha of non-human species as Fc fusion proteins. Non-human interferon-alpha fusion proteins are useful for preclinical studies of interferon-alpha because efficacy and toxicity studies of a protein drug must be performed in animal model systems before testing in human beings. A human protein may not work in a mouse model since the protein may elicit an immune response, and/or exhibit different pharmacokinetics skewing the test results. Therefore, the equivalent mouse protein is the best surrogate for the human protein for testing in a mouse model.

The present invention provides methods of treating various cancers, viral diseases, other diseases, related conditions and causes thereof by administering the DNA, RNA or proteins of the invention to a mammal having such condition. Related conditions may include, but are not limited to, hepatitis B, hepatitis C, hepatitis D, genital warts, hairy-cell leukemia, AIDS-related Kaposi's sarcoma, melanoma, prostate cancer and other forms of viral disease and cancer. In view of the broad roles played by interferon-alpha in modulating immune responses, the present invention also provides methods for treating conditions alleviated by the administration of interferon-alpha. These methods include administering to a mammal having the condition, which may or may not be directly related to viral infection or cancer, an effective amount of a composition of the invention.

The proteins of the invention not only are useful as therapeutic agents, but one skilled in the art recognizes that the proteins are useful in the production of antibodies for diagnostic use. Likewise, appropriate administration of the DNA or RNA, e.g., in a vector or other delivery system for such uses, is included in methods of use of the invention.

As a fusion protein with the immunoglobulin Fc, Fc-Interferon-alpha may have a very favorable tissue distribution and a slightly different mode of action to achieve clinical efficacy, especially in view of its long serum half-life and the high dose of soluble protein that can be administered. In particular, there is a high level of Fc gamma receptor in the liver, which is the site of infection by the viruses causing hepatitis B and hepatitis D. Neurological side effects of interferon-alpha are thought to occur because the small size of interferon-alpha allows it to cross the blood-brain barrier. The much larger size of Fc-Interferon-alpha significantly reduces the extent to which this protein crosses the blood-brain barrier.

Compositions of the present invention may be administered by any route which is compatible with the particular molecules. It is contemplated that the compositions of the present invention may be provided to an animal by any suitable means, directly (e.g., locally, as by injection, implantation or topical administration to a tissue locus) or systemically (e.g., parenterally or orally). Where the composition is to be provided parenterally, such as by intravenous, subcutaneous, ophthalmic, intraperitoneal, intramuscular, buccal, rectal, vaginal, intraorbital, intracerebral, intracranial, intraspinal, intraventricular, intrathecal, intracistemal, intracapsular, intranasal or by aerosol administration, the composition preferably comprises part of an aqueous or physiologically compatible fluid suspension or solution. Thus, the carrier or vehicle is physiologically acceptable so that in addition to delivery of the desired composition to the patient, it does not otherwise adversely affect the patient's electrolyte and/or volume balance. The fluid medium for the agent thus can comprise normal physiologic saline.

The invention features expression vectors for *in vivo* transfection and expression of a fusion protein construct of interferon-alpha in particular cell types so as to reconstitute or supplement the function of interferon-atpha. Expression constructs of fusion protein constructs of interferon-alpha, may be administered in any biologically effective carrier, e.g. any formulation or composition capable of effectively delivering the fusion protein construct of interferon-alpha to cells *in vivo*. Approaches include insertion of the subject gene in viral vectors including recombinant retroviruses, adenovirus, adeno-associated virus, and herpes simplex virus-1, or recombinant bacterial or eukaryotic plasmids.

Preferred dosages of the fusion protein per administration are within the range of 0.1 mg/m² - 100 mg/m², more preferably, 1 mg/m² - 20 mg/m², and most preferably 2 mg/m² - 6 mg/m². It is contemplated that the optimal dosage, however, also depends upon the disease being treated and upon the existence of side effects. However, optimal dosages may be determined using routine experimentation. Administration of the fusion protein may be by periodic bolus injections, or by continuous intravenous or intraperitoneal administration from an external reservoir (for example, from an intravenous bag) or internal (for example, from a bioerodable implant). Furthermore, it is contemplated that the fusion proteins of the invention also may be administered to the intended recipient together with a plurality of different biologically active molecules. It is contemplated, however, that the optimal combination of fusion protein and other molecules, modes of administration, dosages may be determined by routine experimentation well within the level of skill in the art.

The invention is illustrated further by the following non-limiting examples.

### EXAMPLES

### Example 1. Expression of huFc-huInterferon-alpha (huFc-IFN-alpha)

mRNA was prepared from human peripheral blood mononuclear cells and reverse transcribed with reverse transcriptase. The resultant cDNA was used as template for Polymerase Chain Reactions (PCR) to clone and adapt the human interferon-alpha cDNA for expression as a huFc-Interferon-alpha (huFc-IFN-alpha) fusion protein. The forward primer was 5' *C CCG* GGT AAA **TGT GAT CTG CCT CAG AC** (SEQ ID NO: 5), where the sequence *CCCGGG* (XmaI restriction site)TAAA encodes the carboxy terminus of the immunoglobulin heavy chain, followed by sequence (in bold) encoding the N-terminus of interferon-alpha. The reverse primer was *5'CTC GAG* **TCA ATC CTT CCT CCT TAA TC** (SEQ ID NO: 6), which encodes the carboxy-terminal sequence (anti-sense) of interferon-alpha with its translation STOP codon (anticodon, TCA), and this was followed by an Xhol site *(CTCGAG).* A 517 base-pair PCR product was cloned and sequenced. Sequence analysis confirmed that the PCR product encodes mature human Interferon-alpha adapted for expression, i.e., with a Xmal at the 5' end and a XhoI site at the 3' end.

The expression vector pdCs-huFc-IFN-alpha was constructed as follows. The XmaI-XhoI restriction fragment containing the human interferon-alpha cDNA was ligated to the XmaI-XhoI fragment of the pdCs-huFc vector according to Lo *et al.* (1998) *Protein Engineering* 11: 495. huFc is the human Fc fragment of the human immunoglobulin gamma 1. The resultant vector, pdCs-huFc-IFN-alpha, was used to transfect mammalian cells for the expression ofhuFc-IFN-alpha.

### Example 2. Transfection and Expression of Protein

For transient transfection, the plasmid pdCs-huFc-IFN-alpha was introduced into human kidney 293 cells by coprecipitation of plasmid DNA with calcium phosphate (Sambrook et al. eds. (1989) "MOLECULAR CLONING--A LABORATORY MANUAL," Cold Spring Harbor Press, NY) or by lipofection using Lipofectamine Plus (Life Technologies, Gaithersburg, MD) in accordance with the manufacturer's instructions.

In order to obtain stably transfected clones, plasmid DNA was introduced into mouse myeloma NS/0 cells by electroporation. Briefly, NS/0 cells were grown in Dulbecco's modified Eagle's medium supplemented with 10% fetal bovine serum, 2 mM glutamine and penicillin/streptomycin. About 5x10⁶ cells were washed once with phosphate buffered saline (PBS) and resuspended in 0.5 mL PBS. Ten µg of linearized plasmid DNA then was incubated with the cells in a Gene Pulser Cuvette (0.4 cm electrode gap, BioRad) on ice for 10 min. Electroporation was performed using a Gene Pulser (BioRad, Hercules, CA) with settings at 0.25 V and 500 µF. Cells were allowed to recover for 10 min. on ice, after which they were resuspended in growth medium and then plated onto two 96 well plates. Stably transfected clones were selected by growth in the presence of 100 nM methotrexate (MTX), which was introduced two days post-transfection. The cells were fed every 3 days for two to three more times, and MTX-resistant clones appeared in 2 to 3 weeks. Supernatants from clones were assayed by anti-Fc ELISA (see Example 3) to identify high producers. High producing clones were isolated and propagated in growth medium containing 100 nM MTX.

For routine characterization by gel electrophoresis, Fc fusion proteins in the conditioned media were bound to Protein A Sepharose (Repligen, Cambridge, MA) and then eluted from the Protein A Sepharose by boiling in a standard protein sample buffer with or without 2-mercaptoethanol. After electrophoresis on a sodium dodecyl sulfate-polyacrylamide gel electrophoresis (SDS-PAGE), the protein bands were visualized by staining with Coomassie blue. By SDS-PAGE, the huFc-huInterferon-alpha had an apparent MW of about 52 kD.

For purification, the fusion proteins bound on Protein A Sepharose were eluted in a sodium phosphate buffer (100 mM NaH₂P0₄, pH 3, and 150 mM NaCl). The eluate then was immediately neutralized with 0.1 volume of 2 M Tris-hydrochoride, pH 8.

### Example 3. ELISA Procedures

The concentration of human Fc-containing protein products in the supernatants of MTX-resistant clones and other test samples were determined by anti-huFc ELISA. The procedures are described in detail below.

### A. Coating plates.

ELISA plates were coated with AffiniPure Goat anti-Human IgG (H+L) (Jackson Immuno Research Laboratories, West Grove, PA) at 5 µg/mL in PBS, and 100 µL/well in 96-well plates (Nunc-Immuno plate Maxisorp). Coated plates were covered and incubated at 4°C overnight. Plates then were washed 4 times with 0.05% Tween (Tween 20) in PBS, and blocked with 1% BSA/1% goat serum in PBS, 200 µL/well. After incubation with the blocking buffer at 37°C for 2 hrs, the plates were washed 4 times with 0.05% Tween in PBS and tapped dry on paper towels.

### B. Incubation with test samples and secondary antibody

Test samples were diluted as appropriate in sample buffer (1% BSA/1% goat serum/0.05% Tween in PBS). A standard curve was prepared using a chimeric antibody (with a human Fc), the concentration of which was known. To prepare a standard curve, serial dilutions were made in the sample buffer to give a standard curve ranging from 125 ng/mL to 3.9 ng/mL. The diluted samples and standards were added to the plate, 100 µL/well and the plate incubated at 37°C for 2 hr. After incubation, the plate was washed 8 times with 0.05% Tween in PBS. To each well was then added 100 µL of the secondary antibody, the horseradish peroxidase-conjugated anti-human IgG (Jackson Immuno Research), diluted around 1:120,000 in the sample buffer. The exact dilution of the secondary antibody has to be determined for each lot of the HRP-conjugated anti-human IgG. After incubation at 37°C for 2 hr, the plate was washed 8 times with 0.05% Tween in PBS.

### C. Development

The substrate solution was added to the plate at 100 µL/well. The substrate solution was prepared by dissolving 30 mg of OPD (o-phenylenediamine dihydrochloride (OPD), (1 tablet) into 15 mL of 0.025 M Citric acid/0.05 M Na₂HPO₄ buffer, pH 5, which contained 0.03% of freshly added hydrogen peroxide. The color was allowed to develop for 30 min. at room temperature in the dark. The developing time is subject to change, depending on lot to lot variability of the coated plates, the secondary antibody, etc. The reaction was stopped by adding 4N sulfuric acid, 100 µL/well. The plate was read by a plate reader, which was set at both 490 and 650 nm and programmed to subtract the background OD at 650 nm from the OD at 490 nm.

### Example 4. Bioassays

The bioactivity of huFc-huIFN-alpha was compared to that of human interferon-alpha (hu-IFN-alpha) human leucocyte interferon from Sigma, St. Louis, MO) using two different assays. The first assay determines the inhibition of proliferation of Daudi human lymphoblastoid B cell line (ATCC CCL 213). The second assay measures the inhibition of cytopathic effect of encephalomyocarditis virus (EMCV) on human lung carcinoma A549 cell line (ATCC CCL 185).

Interferon-alpha inhibits the proliferation of Daudi (human Burkett lymphoma) cells. Daudi cells were washed with serum-free RPMI 1640 twice, and resuspended in growth medium consisting of RPMI 1640 and 20% heat-inactivated (56°C) fetal bovine serum. The cells then were plated at 1x10⁵ cells/mL/well on a 24-well plate in the presence of different concentrations of αIFH (2.1 x 10⁶ International units/mg) and huFc-huIFN-alpha. After 3-4 days, it was found that 50 pg/mL of IFN-alpha in the form of huFc-huIFN-alpha was as effective as 750 pg/mL of huIFN-alpha in achieving 50-100% inhibition of growth of Daudi cells. As a control, interferon-gamma (Pharmingen, San Diego, CA) at 100 ng/mL showed no activity in this assay. This demonstrates that the inhibition is interferon-alpha specific.

### Example 5. Measurement of Antiviral Activity

Viral replication in cell culture often results in cytotoxicity, an effect known as cytopathic effect (CPE). Interferons can induce an antiviral state in cell cultures and protect cells from such CPE. The antiviral activity IFN-alpha can be quantitated by cytopathic effect reduction (CPER) assays, as described in *"*Lymphokines and Interferons: A Practical Approach," edited by M.J. Clemens, A.G. Morris and A.J.H. Gearing, I.R.L. Press, Oxford, 1987. The antiviral activities of huFc-huIFN-alpha and huIFN-alpha were compared using the human lung carcinoma cell line A549 (ATCC CCL 185) and encephalmyocarditis virus (ATCC VR 129B) according to the CPER protocol described in the above reference. The effective doses to give 50% CPER (i.e., 50% protection) were found to be 570 pg/mL (based on the amount of IFN-alpha) for huFc-huIFN-alpha and 500 pg/mL for huIFN-alpha. Accordingly, the IFN-alpha in huFc-huIFN-alpha and huIFN-alpha have substantially equivalent anti-viral activity.

### Example 6. Pharmacokinetics

The pharmacokinetics of huFc-huIFN-alpha was determined in a group of 4 Balb/c mice. Twenty-five milligrams of huFc-huIFN-alpha was injected into the tail vein of each mouse. Blood was obtained by retro-orbital bleeding immediately after injection (i.e., at t=0 min), and at 0.5, 1, 2, 4, 8 and 24 hr post injection. Blood samples were collected in tubes containing heparin to prevent clotting. Cells were removed by centrifugation in an Eppendorf high-speed microcentrifuge for 4 min. The concentration of huFc-huIFN-alpha in the plasma was measured by anti-huFc ELISA and Western blot analysis with anti-huFc antibody, which also showed that the huFc-huIFN-alpha stayed intact in circulation (52 kD band for huFc-huIFN-alpha). No degradation product (32 kD band for huFc) could be detected. The circulating half-life of huFc-huIFN-alpha was determined to be 19.3 hr, which is significantly longer than the reported circulating half-life of human IFN-alpha of about 2 to 5 hr (PHYSICIANS DESK REFERENCE, 50th edition, 1996:2145-2147 and 2364-2373).

### Example 7. Treatment of Disseminated Growth of Human Burkitt Lymphoma in SCID Mice

Daudi (human Burkitt lymphoma) cells were grown in the C.B-17 SCID (Severe Combined Immune Deficiency) mice as disseminated tumors (Ghetie et al. (1990) INTL. J. CANCER: 45:481). About 5x10⁶ Daudi cells of a single cells suspension in 0.2 mL PBSB were injected intravenously into 6-8 week old SCID mice. Three days later, mice were randomized into three groups of eight and received daily intraperitoneal injections of 0.2 mL of PBS, 30 µg of huFc-huIFN-alpha (containing about 12 µg of IFN-alpha) in PBS, or 60 µg of huFc-huIFN-alpha in PBS. Mice were monitored daily. The results are presented in Figure 2.

By Day 28 after the Daudi cell injection, all mice in the control PBS (diamonds) group had developed paralysis of the hind legs. Mice in this PBS control group began dying on Day 38 and by Day 61, all the mice in the control group died. In contrast, the mice in the treatment groups survived much longer, and in a dose-dependent manner. For the group that received 30 µg of huFc-huIFN-alpha (crosses), the first death occurred on Day 70, and all mice died by Day 134. Four the group that received 60 µg of huFc-huIFN-alpha (triangles), the first death did not occur till Day 126, and four more died on Day 153. The rest of the mice were sick and were euthanized.

### Example 8. Treatment of Localized Growth of Human Burkett Lymphoma in SCID mice.

In this model, Daudi cells were grown in the C.B-17 SCID mice as subcutaneous tumors (*Ghetie et al.* (1990) INT. J. CANCER: 45-481). About 6x10⁶ Daudi cells of a single cell suspension in 0.1 mL PBS were injected subcutaneously into 6-8 week old SCID mice. Treatment started when the tumor size reached 200-400 mm³, which took about 4 weeks. Mice were randomized into 3 groups of 8, and each groups received 6 daily intraperitoneal injections of 0.2 mL of PBS, 30 µg of huFc-huIFN-alpha in PBS, or 60 µg of huFc-huIFN-alpha in PBS. The results are shown in Figure 3. Size of tumors was measured twice a week.

The tumors in the control group mice (diamonds) grew rapidly to a mean volume of 5602 mm³ (range: 4343-6566 mm³) by day 35, after which all the mice in the group were euthanized. In contrast, the growth of tumors in the mice in the treatment groups were suppressed in a dose-dependent manner. The groups that received 30 µg and 60 µg of huFc-huIFN-alpha had mean tumor volumes of 214 and 170 mm³, respectively, at day 35, which were smaller than the 268 and 267 mm³ before treatment. In fact, the subcutaneous tumors had completely shrunk in 5 out of 8 mice in the group receiving 30 µg huFc-huIFN-alpha, and 4 out of 8 mice in the group receiving 60 µg of huFc-huIFN-alpha. Without further treatment, however, some of the tumors did return and grew. Nevertheless, two mice in the group remained tumor-free until day 205, when the experiment was terminated.

### Example 9. Treatment of Liver Disease with Fc-Interferon-alpha.

It is contemplated that a liver disease, for example, hepatitis or liver metastases, can be treated more effectively with Fc-Interferon-alpha than with interferon-alpha or interferon-alpha-Fc.

For example, it is contemplated that Fc-interferon-alpha can be effective in treating a mouse model in which tumor cells metastasize to the liver. Mice are anaesthetized by intraperitoneal injection of 80 mg/kg ketamine and 5 mg/kg xylazine in 0.2 ml PBS about 5 minutes before surgery. The following steps then are performed in a laminar flow hood to ensure sterility. The skin of each mouse is cleaned with betadine and ethanol Tumor cells, such as Daudi cells, are injected in 100 microliters of RPMI 1640medium without supplement beneath the splenic capsule over a period of about one minute using a 27-gauge needle. After two minutes, the splenic pedicle is ligated with a 4.0 silk suture and the spleen is removed.

Some cells are carried from the site of injection into the liver, where they can form metastatic tumors. Mice with metastatic liver tumors then are treated with Fc-interferon-alpha. It is contemplated that mice treated with Fc-interferon-alpha show a significant reduction in tumor growth relative to mice treated with an equimolar amount of interferon-alpha or interferon-alpha-Fc fusion protein.

Furthermore, it is contemplated that the specific effect of Fc-interferon-alpha is more pronounced in treatment of liver disease than in treatment of disorders localized to other tissues where Fc-interferon-alpha is not concentrated.

## Claims

1. A fusion protein having the biological activity of interferon-alpha and being able to concentrate interferon-alpha in the liver, said fusion protein comprising in N-to C-terminal direction an immunoglobulin Fc region deriving from IgG1 or IgG3 and a target protein comprising interferon-alpha.

2. A fusion protein according to claim 1, wherein the Fc region comprises at least an immunoglobulin hinge region, a CH2 domain and a CH3 domain.

3. A fusion protein according to claim 1 or 2, wherein the target protein comprises a second full-length interferon-alpha or a bioactive fragment thereof.

4. A fusion protein according to claim 3, wherein the first and the second interferon-alpha protein are linked together directly or by means of a polypeptide linker.

5. A fusion protein according to claim 3 or 4, wherein the Fc region and the target protein are linked together directly or by means of a polypeptide linker.

6. A multimeric fusion protein comprising at last two fusion proteins as specified in claims 1 to 5 associated by means of a covalent bond.

7. A DNA molecule encoding a fusion protein accordimg to any of the claims 1 to 6.

8. A DNA molecule of claim 7, wherein the fusion protein comprises a signal sequence, said signal sequence, said Fc region and said target protein are encoded serially in a 5' to 3' direction.

9. A pharmaceutical composition comprising a fusion protein according to any of the claims 1 to 6 optionally tohether with a pharmaceutically acceptable carrier.

10. Use of a fusion protein according to any of the claims 1 to 6 or a pharmaceutical composition of claim 9 for the manufacture of a medicament for the treatment of liver diseases.

11. Use of a fusion protein according to claim 10, wherein the liver disease is hepatitis..

## Patentansprüche

1. Fusionsprotein mit der biologischen Aktivität von Interferon alpha, das Interferon alpha in der Leber anreichern kann, wobei dieses Fusionsprotein in Richtung vom N-zum C-Terminus eine Immunglobulin-Fc-Region, die von IgG1 oder IgG3 abstammt, und ein Zielprotein, enthaltend Interferon alpha, enthält.

2. Fusionsprotein nach Anspruch 1, wobei die Fc-Region mindestens eine Gelenk-Region des Immunglobulins, eine CH2-Domäne und eine CH3-Domäne enthält.

3. Fusionsprotein nach Anspruch 1 oder 2, wobei das Zielprotein ein zweites Volllängen-Interferon-alpha oder ein biologisch aktives Fragment davon enthält.

4. Fusionsprotein nach Anspruch 3, wobei das erste und das zweite Interferon-alpha-Protein direkt oder mittels eines Polypeptidlinkers miteinander verbunden sind.

5. Fusionsprotein nach Anspruch 3 oder 4, wobei die Fc-Region und das Zielprotein direkt oder mittels eines Polypeptidlinkers miteinander verbunden sind.

6. Multimeres Fusionsprotein, enthaltend schließlich zwei Fusionsproteine wie in den Ansprüchen 1 bis 5 spezifiziert, verknüpft mittels einer kovalenten Bindung.

7. DNA-Molekül, kodierend für ein Fusionsprotein nach einem der Ansprüche 1 bis 6.

8. DNA-Molekül nach Anspruch 7, wobei das Fusionsprotein eine Signalsequenz enthält, wobei diese Signalsequenz, diese Fc-Region und dieses Zielprotein nacheinander in einer Richtung von 5' nach 3' kodiert werden.

9. Pharmazeutische Zusammensetzung, enthaltend ein Fusionsprotein nach einem der Ansprüche 1 bis 6, gegebenenfalls zusammen mit einem pharmazeutisch unbedenklichen Träger.

10. Verwendung eines Fusionsproteins nach einem der Ansprüche 1 bis 6 oder einer pharmazeutischen Zusammensetzung nach Anspruch 9 zur Herstellung eines Arzneimittels zur Behandlung von Lebererkrankungen.

11. Verwendung eines Fusionsproteins nach Anspruch 10, wobei die Lebererkrankung Hepatitis ist.

## Revendications

1. Protéine de fusion présentant l'activité biologique de l'interféron-alpha et pouvant concentrer l'interféron-alpha dans le foie; ladite protéine de fusion comprenant dans la direction de terminus N vers C une région Fc d'immunoglobuline dérivant à partir d'IgG1 ou d'IgG3 et une protéine cible comprenant l'interféron-alpha.

2. Protéine de fusion selon la revendication 1, dans laquelle la région Fc comprend au moins une région de charnière d'immunoglobuline, un domaine CH2 et un domaine CH3.

3. Protéine de fusion selon la revendication 1 ou 2, dans laquelle la protéine cible comprend un second interféron-alpha pleine longueur ou son fragment bioactif.

4. Protéine de fusion selon la revendication 3, dans laquelle les première et seconde protéines d'interféron-alpha sont liées ensemble directement ou au moyen d'un lieur polypeptidique.

5. Protéine de fusion selon la revendication 3 ou 4, dans laquelle la région Fc et la protéine cible sont liées ensemble directement ou au moyen d'un lieur polypeptidique.

6. Protéine de fusion multimérique comprenant au moins deux protéines de fusion telles que spécifiées selon les revendications 1 à 5 associées au moyen d'une liaison covalente.

7. Molécule d'ADN codant une protéine de fusion selon l'une quelconque des revendications 1 à 6.

8. Molécule d'ADN selon la revendication 7, dans laquelle la protéine de fusion comprend une séquence signal, ladite séquence signal, ladite région Fc et ladite protéine cible sont codées série dans une direction 5' vers 3'.

9. Composition pharmaceutique comprenant une protéine de fusion selon l'une quelconque des revendications 1 à 6 en option en association avec un support pharmaceutiquement acceptable.

10. Utilisation d'une protéine de fusion selon l'une quelconque des revendications 1 à 6 ou d'une composition pharmaceutique selon la revendication 9 pour la fabrication d'un médicament pour le traitement de maladies du foie.

11. Utilisation d'une protéine de fusion selon la revendication 10, dans laquelle la maladie du foie est l'hépatite.


### SEQUENCE LISTING

<110> Lo, Kin-Ming
   Sun, Yaping
   Gillies, Stephen D.
   Lexigen Pharmaceuticals Corp.
<120> Expression and Export of Interferon-Alpha Proteins as Fc Fusion Proteins
<130> LEX-009 PC
<140>
   <141>
<150> US 60/134,895
   <151> 1999-05-19
<160> 29
<170> Patent In Ver. 2.0
<210> 1
   <211> 498
   <212> DNA
   <213> Homo sapiens
<220>
   <221> CDS
   <222> (1)..(498)
   <223> Human IFN alpha DNA sequence
<400> 1
   tgt gat ctg cct cag acc cac agc ctg ggt aat agg agg gcc ttg ata    48
   Cys Asp Leu Pro Gln Thr His Ser Leu Gly Asn Arg Arg Ala Leu Ile
    1               5                  10                  15
   ctc ctg gca caa atg gga aga atc tct cct ttc tcc tgc ctg aag gac    96
   Leu Leu Ala Gln Met Gly Arg Ile Ser Pro Phe Ser Cys Leu Lys Asp
               20                  25                  30
   aga cat gac ttt gga ttc ccc cag gag gag ttt gat ggc aac cag ttc   144
   Arg His Asp Phe Gly Phe Pro Gln Glu Glu Phe Asp Gly Asn Gln Phe
           35                  40                  45
   cag aag gct caa gcc atc cct gtc ctc cat gag atg atc cag cag acc   192
   Gln Lys Ala Gln Ala Ile Pro Val Leu His Glu Met Ile Gln Gln Thr
       50                  55                  60
   ttc aat ctc ttc agc aca aag gac tca tct gct act tgg gaa cag agc   240
   Phe Asn Leu Phe Ser Thr Lys Asp Ser Ser Ala Thr Trp Glu Gln Ser
   65                  70                  75                  80
   ctc cta gaa aaa ttt tcc act gaa ctt aac cag cag ctg aat gac ctg   288
   Leu Leu Glu Lys Phe Ser Thr Glu Leu Asn Gln Gln Leu Asn Asp Leu
                   85                  90                  95
   gaa gcc tgc gtg ata cag gag gtt ggg gtg gaa gag act ccc ctg atg   336
   Glu Ala Cys Val Ile Gln Glu Val Gly Val Glu Glu Thr Pro Leu Met
               100                 105                 110
   aat gtg gac tcc atc ctg gct gtg aag aaa tac ttc caa aga atc act   384
   Asn Val Asp Ser Ile Leu Ala Val Lys Lys Tyr Phe Gln Arg Ile Thr
           115                 120                 125
   ctt tat ctg aca gag aag aaa tac agc cct tgt gcc tgg gag gtt gtc   432
   Leu Tyr Leu Thr Glu Lys Lys Tyr Ser Pro Cys Ala Trp Glu Val Val
       130                 135                 140
   aga gca gaa atc atg aga tcc ttc tct tta tca aaa att ttt caa gaa   480
   Arg Ala Glu Ile Met Arg Ser Phe Ser Leu Ser Lys Ile Phe Gln Glu
   145                 150                 155                 160
   aga tta agg aag aag gat                                           498
   Arg Leu Arg Lys Lys Asp
                   165
<210> 2
   <211> 166
   <212> PRT
   <213> Homo sapiens
<400> 2
   Cys Asp Leu Pro Gln Thr His Ser Leu Gly Asn Arg Arg Ala Leu Ile
   1               5                   10                  15
   Leu Leu Ala Gln Met Gly Arg Ile Ser Pro Phe Ser Cys Leu Lys Asp
               20                  25                  30
   Arg His Asp Phe Gly Phe Pro Gln Glu Glu Phe Asp Gly Asn Gln Phe
           35                  40                  45
   Gln Lys Ala Gln Ala Ile Pro Val Leu His Glu Met Ile Gln Gln Thr
       50                  55                  60
   Phe Asn Leu Phe Ser Thr Lys Asp Ser Ser Ala Thr Trp Glu Gln Ser
   65                  70                  75                  80
   Leu Leu Glu Lys Phe Ser Thr Glu Leu Asn Gln Gln Leu Asn Asp Leu
                   85                  90                  95
   Glu Ala Cys Val Ile Gln Glu Val Gly Val Glu Glu Thr Pro Leu Met
               100                 105                 110
   Asn Val Asp Ser Ile Leu Ala Val Lys Lys Tyr Phe Gln Arg Ile Thr
           115                 120                 125
   Leu Tyr Leu Thr Glu Lys Lys Tyr Ser Pro Cys Ala Trp Glu Val Val
       130                 135                 140
   Arg Ala Glu Ile Met Arg Ser Phe Ser Leu Ser Lys Ile Phe Gln Glu
   145                 150                 155                 160
   Arg Leu Arg Lys Lys Asp
                   165
<210> 3
   <211> 696
   <212> DNA
   <213> Homo sapiens
<220>
   <221> CDS
   <222> (1) .. (696)
   <223> Human Fc DNA sequence
<400> 3
   gag ccc aaa tct tct gac aaa act cac aca tgc cca ccg tgc cca gca    48
   Glu Pro Lys Ser Ser Asp Lys Thr His Thr Cys Pro Pro Cys Pro Ala
    1               5                  10                  15
   cct gaa ctc ctg ggg gga ccg tca gtc ttc ctc ttc ccc cca aaa ccc    96
   Pro Glu Leu Leu Gly Gly Pro Ser Val Phe Leu Phe Pro Pro Lys Pro
               20                  25                  30
   aag gac acc ctc atg atc tcc cgg acc cct gag gtc aca tgc gtg gtg   144
   Lys Asp Thr Leu Met Ile Ser Arg Thr Pro Glu Val Thr Cys Val Val
           35                  40                  45
   gtg gac gtg agc cac gaa gac cct gag gtc aag ttc aac tgg tac gtg   192
   Val Asp Val Ser His Glu Asp Pro Glu Val Lys Phe Asn Trp Tyr Val
       50                  55                  60
   gac ggc gtg gag gtg cat aat gcc aag aca aag ccg cgg gag gag cag   240
   Asp Gly Val Glu Val His Asn Ala Lys Thr Lys Pro Arg Glu Glu Gln
   65                  70                  75                  80
   tac aac agc acg tac cgt gtg gtc agc gtc ctc acc gtc ctg cac cag   288
   Tyr Asn Ser Thr Tyr Arg Val Val Ser Val Leu Thr Val Leu His Gln
                   85                  90                  95
   gac tgg ctg aat ggc aag gag tac aag tgc aag gtc tcc aac aaa gcc   336
   Asp Trp Leu Asn Gly Lys Glu Tyr Lys Cys Lys Val Ser Asn Lys Ala
               100                 105                 110
   ctc cca gcc ccc atc gag aaa acc atc tcc aaa gcc aaa ggg cag ccc   384
   Leu Pro Ala Pro Ile Glu Lys Thr Ile Ser Lys Ala Lys Gly Gln Pro
           115                 120                 125
   cga gaa cca cag gtg tac acc ctg ccc cca tca cgg gag gag atg acc   432
   Arg Glu Pro Gln Val Tyr Thr Leu Pro Pro Ser Arg Glu Glu Met Thr
       130                 135                 140
   aag aac cag gtc agc ctg acc tgc ctg gtc aaa ggc ttc tat ccc agc   480
   Lys Asn Gln Val Ser Leu Thr Cys Leu Val Lys Gly Phe Tyr Pro Ser
   145                 150                 155                 160
   gac atc gcc gtg gag tgg gag agc aat ggg cag ccg gag aac aac tac   528
   Asp Ile Ala Val Glu Trp Glu Ser Asn Gly Gln Pro Glu Asn Asn Tyr
                   165                 170                 175
   aag acc acg cct ccc gtg ctg gac tcc gac ggc tcc ttc ttc ctc tat   576
   Lys Thr Thr Pro Pro Val Leu Asp Ser Asp Gly Ser Phe Phe Leu Tyr
               180                 185                 190
   agc aag ctc acc gtg gac aag agc agg tgg cag cag ggg aac gtc ttc   624
   Ser Lys Leu Thr Val Asp Lys Ser Arg Trp Gln Gln Gly Asn Val Phe
           195                 200                 205
   tca tgc tcc gtg atg cat gag gct ctg cac aac cac tac acg cag aag   672
   Ser Cys Ser Val Met His Glu Ala Leu His Asn His Tyr Thr Gln Lys
       210                 215                 220
   agc ctc tcc ctg tcc ccg ggt aaa                                         696
   Ser Leu Ser Leu Ser Pro Gly Lys
   225                 230
<210> 4
   <211> 232
   <212> PRT
   <213> Homo sapiens
<400> 4
   Glu Pro Lys Ser Ser Asp Lys Thr His Thr Cys Pro Pro Cys Pro Ala
    1               5                  10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Glu Glu Met Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 5
   <211> 27
   <212> DNA
   <213> Homo sapiens
<220>
   <223> Forward PCR primer
<400> 5
   cccgggtaaa tgtgatctgc ctcagac 27
<210> 6
   <211> 26
   <212> DNA
   <213> Homo sapiens
<400> 6
   ctcgagtcaa tccttcctcc ttaatc 26
<210> 7
   <211> 162
   <212> PRT
   <213> Homo sapiens
<220>
   <223> IFN alpha consensus sequence wherein, Xaa at any position besides positions 24,31,70 and 129 represents any amino acid.
<220>
   <223> Xaa24 can be Ile or Leu, Xaa31 can be Lys or Gln, Xaa70 can be Thr or Ser and Xaa 129 can be Leu or Val.
<400> 7
   Cys Asp Leu Xaa Xaa Xaa Xaa Xaa Leu Xaa Xaa Xaa Xaa Xaa Leu Xaa
   1               5                   10                  15
   Xaa Xaa Xaa Xaa Met Xaa Xaa Xaa Ser Pro Xaa Xaa Cys Leu Xaa Xaa
               20                  25                  30
   Arg Xaa Asp Phe Xaa Xaa Pro Xaa Glu Xaa Xaa Xaa Xaa Xaa Gln Xaa
           35                  40                  45
   Xaa Xaa Xaa Gln Ala Xaa Xaa Val Leu Xaa Xaa Xaa Xaa Gln Gln Xaa
       50                  55                  60
   Xaa Xaa Leu Phe Xaa Xaa Xaa Xaa Xaa Ser Ala Xaa Trp Xaa Xaa Thr
   65                  70                  75                  80
   Leu Leu Xaa Xaa Xaa Xaa Xaa Xaa Leu Xaa Gln Gln Leu Xaa Asp Leu
                   85                  90                  95
   Xaa Xaa Cys Xaa Xaa Xaa Xaa Xaa Xaa Xaa Xaa Xaa Xaa Xaa Leu Xaa
               100                 105                 110
   Xaa Val Xaa Xaa Xaa Leu Xaa Val Xaa Xaa Tyr Phe Xaa Xaa Ile Xaa
           115                 120                 125
   Xaa Tyr Leu Xaa Xaa Lys Xaa Xaa Ser Xaa Cys Ala Trp Glu Xaa Xaa
       130                 135                 140
   Xaa Xaa Xaa Xaa Met Arg Xaa Xaa Ser Xaa Xaa Xaa Xaa Leu Xaa Xaa
   145                 150                 155                 160
   Arg Leu
<210> 8
   <211> 166
   <212> PRT
   <213> Homo sapiens
<220>
   <223> Human IFN alpha-1 protein
<400> 8
   Cys Asp Leu Pro Glu Thr His Ser Leu Asp Asn Arg Arg Thr Leu Met
   1 5 10 15
   Leu Leu Ala Gln Met Ser Arg Ile Ser Pro Ser Ser Cys Leu Met Asp
   20 25 30
   Arg His Asp Phe Gly Phe Pro Gln Glu Glu Phe Asp Gly Asn Gln Phe
   35 40 45
   Gln Lys Ala Pro Ala Ile Ser Val Leu His Glu Leu Ile Gln Gln Ile
   50 55 60
   Phe Asn Leu Phe Thr Thr Lys Asp Ser Ser Ala Ala Trp Asp Glu Asp
   65 70 75 80
   Leu Leu Asp Lys Phe Cys Thr Glu Leu Tyr Gln Gln Leu Asn Asp Leu
   85 90 95
   Glu Ala Cys Val Met Gln Glu Glu Arg Val Gly Glu Thr Pro Leu Met
   100 105 110
   Asn Ala Asp Ser Ile Leu Ala Val Lys Lys Tyr Phe Arg Arg Ile Thr
   115 120 125
   Leu Tyr Leu Thr Glu Lys Lys Tyr Ser Pro Cys Ala Trp Glu Val Val
   130 135 140
   Arg Ala Glu Ile Met Arg Ser Leu Ser Leu Ser Thr Asn Leu Gln Glu
   145 150 155 160
   Arg Leu Arg Arg Lys Glu
   165
<210> 9
   <211> 165
   <212> PRT
   <213> Homo sapiens
<220>
   <223> Human IFN alpha-2 protein
<400> 9
   Cys Asp Leu Pro Gln Thr His Ser Leu Gly Ser Arg Arg Thr Leu Met
   1               5                   10                  15
   Leu Leu Ala Gln Met Arg Lys Ile Ser Leu Phe Ser Cys Leu Lys Asp
               20                  25                  30
   Arg His Asp Phe Gly Phe Pro Gln Glu Glu Phe Gly Asn Gln Phe Gln
           35                  40                  45
   Lys Ala Glu Thr Ile Pro Val Leu His Glu Met Ile Gln Gln Ile Phe
       50                  55                  60
   Asn Leu Phe Ser Thr Lys Asp Ser Ser Ala Ala Trp Asp Glu Thr Leu
   65                  70                  75                  80
   Leu Asp Lys Phe Tyr Thr Glu Leu Tyr Gln Gln Leu Asn Asp Leu Glu
                   85                  90                  95
   Ala Cys Val Ile Gln Gly Val Gly Val Thr Glu Thr Pro Leu Met Lys
               100                 105                 110
   Glu Asp Ser Ile Leu Ala Val Arg Lys Tyr Phe Gln Arg Ile Thr Leu
           115                 120                 125
   Tyr Leu Lys Glu Lys Lys Tyr Ser Pro Cys Ala Trp Glu Val Val Arg
       130                 135                 140
   Ala Glu Ile Met Arg Ser Phe Ser Leu Ser Thr Asn Leu Gln Glu Ser
   145                 150                 155                 160
   Leu Arg Ser Lys Glu
                   165
<210> 10
   <211> 166
   <212> PRT
   <213> Homo sapiens
<220>
   <223> Human IFN alpha-4 protein
<400> 10
   Cys Asp Leu Pro Gln Thr His Ser Leu Gly Asn Arg Arg Ala Leu Ile
    1               5                  10                  15
   Leu Leu Ala Gln Met Gly Arg Ile Ser His Phe Ser Cys Leu Lys Asp
               20                  25                  30
   Arg His Asp Phe Gly Phe Pro Glu Glu Glu Phe Asp Gly His Gln Phe
           35                  40                  45
   Gln Lys Thr Gln Ala Ile Ser Val Leu His Glu Met Ile Gln Gln Thr
       50                  55                  60
   Phe Asn Leu Phe Ser Thr Glu Asp Ser Ser Ala Ala Trp Glu Gln Ser
   65                  70                  75                      80
   Leu Leu Glu Lys Phe Ser Thr Glu Leu Tyr Gln Gln Leu Asn Asp Leu
                   85                  90                  95
   Glu Ala Cys Val Ile Gln Glu Val Gly Val Glu Glu Thr Pro Leu Met
               100                 105                 110
   Asn Val Asp Ser Ile Leu Ala Val Arg Lys Tyr Phe Gln Arg Ile Thr
           115                 120                 125
   Leu Tyr Leu Thr Glu Lys Lys Tyr Ser Pro Cys Ala Trp Glu Val Val
       130                 135                 140
   Arg Ala Glu Ile Met Arg Ser Leu Ser Phe Ser Thr Asn Leu Gln Lys
   145                 150                 155                     160
   Arg Leu Arg Arg Lys Asp
                   165
<210> 11
   <211> 166
   <212> PRT
   <213> Homo sapiens
<220>
   <223> Human IFN alpha-5 protein
<400> 11
   Cys Asp Leu Pro Gln Thr His Ser Leu Ser Asn Arg Arg Thr Leu Met
   1               5                   10                  15
   Ile Met Ala Gln Met Gly Arg Ile Ser Pro Phe Ser Cys Leu Lys Asp
               20                  25                  30
   Arg His Asp Phe Gly Phe Pro Gln Glu Glu Phe Asp Gly Asn Gln Phe
           35                  40                  45
   Gln Lys Ala Gln Ala Ile Ser Val Leu His Glu Met Ile Gln Gln Thr
       50                  55                  60
   Phe Asn Leu Phe Ser Thr Lys Asp Ser Ser Ala Thr Trp Asp Glu Thr
   65                  70                  75                  80
   Leu Leu Asp Lys Phe Tyr Thr Glu Leu Tyr Gln Gln Leu Asn Asp Leu
                   85                  90                  95
   Glu Ala Cys Met Met Gln Glu Val Gly Val Glu Asp Thr Pro Leu Met
               100                 105                 110
   Asn Val Asp Ser Ile Leu Thr Val Arg Lys Tyr Phe Gln Arg Ile Thr
           115                 120                 125
   Leu Tyr Leu Thr Glu Lys Lys Tyr Ser Pro Cys Ala Trp Glu Val Val
       130                 135                 140
   Arg Ala Glu Ile Met Arg Ser Phe Ser Leu Ser Ala Asn Leu Gln Glu
   145                 150                 155                 160
   Arg Leu Arg Arg Lys Glu
                   165
<210> 12
   <211> 166
   <212> PRT
   <213> Homo sapiens
<220>
   <223> HUman IFN alpha-6 protein
<400> 12
   Cys Asp Leu Pro Gln Thr His Ser Leu Gly His Arg Arg Thr Met Met
   1 5 10 15
   Leu Leu Ala Gln Met Arg Arg Ile Ser Leu Phe Ser Cys Leu Lys Asp
   20 25 30
   Arg His Asp Phe Arg Phe Pro Gln Glu Glu Phe Asp Gly Asn Gln Phe
   35 40 45
   Gln Lys Ala Glu Ala Ile Ser Val Leu His Glu Val Ile Gln Gln Thr
   50 55 60
   Phe Asn Leu Phe Ser Thr Lys Asp Ser Ser Val Ala Trp Asp Glu Arg
   65 70 75 80
   Leu Leu Asp Lys Leu Tyr Thr Glu Leu Tyr Gln Gln Leu Asn Asp Leu
   85 90 95
   Glu Ala Cys Val Met Gln Glu Val Trp Val Gly Gly Thr Pro Leu Met
   100 105 110
   Asn Glu Asp Ser Ile Leu Ala Val Arg Lys Tyr Phe Gln Arg Ile Thr
   115 120 125
   Leu Tyr Leu Thr Glu Lys Lys Tyr Ser Pro Cys Ala Trp Glu Val Val
   130 135 140
   Arg Ala Glu Ile Met Arg Ser Phe Ser Ser Ser Arg Asn Leu Gln Glu
   145 150 155 160
   Arg Leu Arg Arg Lys Glu
   165
<210> 13
   <211> 166
   <212> PRT
   <213> Homo sapiens
<220>
   <223> Human IFN alpha-7 protein
<400> 13
   Cys Asp Leu Pro Gln Thr His Ser Leu Arg Asn Arg Arg Ala Leu Ile
   1 5 10 15
   Leu Leu Ala Gln Met Gly Arg Ile Ser Pro Phe Ser Cys Leu Lys Asp
   20 25 30
   Arg His Glu Phe Arg Phe Pro Glu Glu Glu Phe Asp Gly His Gln Phe
   35 40 45
   Gln Lys Thr Gln Ala Ile Ser Val Leu His Glu Met Ile Gln Gln Thr
   50 55 60
   Phe Asn Leu Phe Ser Thr Glu Asp Ser Ser Ala Ala Trp Glu Gln Ser
    65                  70                  75                  80
   Leu Leu Glu Lys Phe Ser Thr Glu Leu Tyr Gln Gln Leu Asn Asp Leu
                    85                  90                  95
   Glu Ala Cys Val Ile Gln Glu Val Gly Val Glu Glu Thr Pro Leu Met
               100                 105                 110
   Asn Glu Asp Phe Ile Leu Ala Val Arg Lys Tyr Phe Gln Arg Ile Thr
           115                 120                 125
   Leu Tyr Leu Met Glu Lys Lys Tyr Ser Pro Cys Ala Trp Glu Val Val
       130                 135                 140
   Arg Ala Glu Ile Met Arg Ser Phe Ser Phe Ser Thr Asn Leu Lys Lys
   145                 150                 155                 160
   Gly Leu Arg Arg Lys Asp
                   165
<210> 14
   <211> 166
   <212> PRT
   <213> Homo sapiens
<220>
   <223> Human IFN alpha-8 protein
<400> 14
   Cys Asp Leu Pro Gln Thr His Ser Leu Gly Asn Arg Arg Ala Leu Ile
   1 5 10 15
   Leu Leu Ala Gln Met Arg Arg Ile Ser Pro Phe Ser Cys Leu Lys Asp
   20 25 30
   Arg His Asp Phe Glu Phe Pro Gln Glu Glu Phe Asp Asp Lys Gln Phe
   35 40 45
   Gln Lys Ala Gln Ala Ile Ser Val Leu His Glu Met Ile Gln Gln Thr
   50 55 60
   Phe Asn Leu Phe Ser Thr Lys Asp Ser Ser Ala Ala Leu Asp Glu Thr
   65 70 75 80
   Leu Leu Asp Glu Phe Tyr Ile Glu Leu Asp Gln Gln Leu Asn Asp Leu
   85 90 95
   Glu Val Leu Cys Asp Gln Glu Val Gly Val Ile Glu Ser Pro Leu Met
   100 105 110
   Tyr Glu Asp Ser Ile Leu Ala Val Arg Lys Tyr Phe Gln Arg Ile Thr
   115 120 125
   Leu Tyr Leu Thr Glu Lys Lys Tyr Ser Ser Cys Ala Trp Glu Val Val
   130 135 140
   Arg Ala Glu Ile Met Arg Ser Phe Ser Leu Ser Ile Asn Leu Gln Lys
   145 150 155 160
   Arg Leu Lys Ser Lys Glu
   165
<210> 15
   <211> 166
   <212> PRT
   <213> Homo sapiens
<220>
   <223> Human IFN alpha-10 protein
<400> 15
   Cys Asp Leu Pro Gln Thr His Ser Leu Gly Asn Arg Arg Ala Leu Ile
   1 5 10 15
   Leu Leu Gly Gln Met Gly Arg Ile Ser Pro Phe Ser Cys Leu Lys Asp
   20 25 30
   Arg His Asp Phe Arg Ile Pro Gln Glu Glu Phe Asp Gly Asn Gln Phe
   35 40 45
   Gln Lys Ala Gln Ala Ile Ser Val Leu His Glu Met Ile Gln Gln Thr
   50 55 60
   Phe Asn Leu Phe Ser Thr Glu Asp Ser Ser Ala Ala Trp Glu Gln Ser
   65 70 75 80
   Leu Leu Glu Lys Phe Ser Thr Glu Leu Tyr Gln Gln Leu Asn Asp Leu
   85 90 95
   Glu Ala Cys Val Ile Gln Glu Val Gly Val Glu Glu Thr Pro Leu Met
   100 105 110
   Asn Glu Asp Ser Ile Leu Ala Val Arg Lys Tyr Phe Gln Arg Ile Thr
   115 120 125
   Leu Tyr Leu Ile Glu Arg Lys Tyr Ser Pro Cys Ala Trp Glu Val Val
   130 135 140
   Arg Ala Glu Ile Met Arg Ser Leu Ser Phe Ser Thr Asn Leu Gln Lys
   145 150 155 160
   Arg Leu Arg Arg Lys Asp
   165
<210> 16
   <211> 170
   <212> PRT
   <213> Homo sapiens
<220>
   <223> Human IFN alpha-14 protein
<400> 16
   Cys Ser Leu Gly Cys Asn Leu Ser Gln Thr His Ser Leu Asn Asn Arg
   1 5 10 15
   Arg Thr Leu Met Leu Met Ala Gln Met Arg Arg Ile Ser Pro Phe Ser
   20 25 30
   Cys Leu Lys Asp Arg His Asp Phe Glu Phe Pro Gln Glu Glu Phe Asp
           35                  40                  45
   Gly Asn Gln Phe Gln Lys Ala Gln Ala Ile Ser Val Leu His Glu Met
       50                  55                  60
   Met Gln Gln Thr Phe Asn Leu Phe Ser Thr Lys Asn Ser Ser Ala Ala
   65                  70                  75                  80
   Trp Asp Glu Thr Leu Leu Glu Lys Phe Tyr Ile Glu Leu Phe Gln Gln
                   85                  90                  95
   Met Asn Asp Leu Glu Ala Cys Val Ile Gln Glu Val Gly Val Glu Glu
               100                 105                 110
   Thr Pro Leu Met Asn Glu Asp Ser Ile Leu Ala Val Lys Lys Tyr Phe
           115                 120                 125
   Gln Arg Ile Thr Leu Tyr Leu Met Glu Lys Lys Tyr Ser Pro Cys Ala
       130                 135                 140
   Trp Glu Val Val Arg Ala Glu Ile Met Arg Ser Phe Ser Phe Ser Thr
   145                 150                 155                 160
   Asn Leu Gln Lys Arg Leu Arg Arg Lys Asp
                   165                 170
<210> 17
   <211> 166
   <212> PRT
   <213> Homo sapiens
<220>
   <223> Human IFN alpha-16 protein
<400> 17
   Cys Asp Leu Pro Gln Thr His Ser Leu Gly Asn Arg Arg Ala Leu Ile
    1               5                  10                  15
   Leu Leu Ala Gln Met Gly Arg Ile Ser His Phe Ser Cys Leu Lys Asp
               20                  25                  30
   Arg Tyr Asp Phe Gly Phe Pro Gln Glu Val Phe Asp Gly Asn Gln Phe
           35                  40                  45
   Gln Lys Ala Gln Ala Ile Ser Ala Phe His Glu Met Ile Gln Gln Thr
       50                  55                  60
   Phe Asn Leu Phe Ser Thr Lys Asp Ser Ser Ala Ala Trp Asp Glu Thr
   65                  70                  75                  80
   Leu Leu Asp Lys Phe Tyr Ile Glu Leu Phe Gln Gln Leu Asn Asp Leu
                   85                  90                  95
   Glu Ala Cys Val Thr Gln Glu Val Gly Val Glu Glu Ile Ala Leu Met
               100                 105                 110
   Asn Glu Asp Ser Ile Leu Ala Val Arg Lys Tyr Phe Gln Arg Ile Thr
           115                 120                 125
   Leu Tyr Leu Met Gly Lys Lys Tyr Ser Pro Cys Ala Trp Glu Val Val
       130                 135                 140
   Arg Ala Glu Ile Met Arg Ser Phe Ser Phe Ser Thr Asn Leu Gln Lys
   145                 150                 155                 160
   Gly Leu Arg Arg Lys Asp
                   165
<210> 18
   <211> 166
   <212> PRT
   <213> Homo sapiens
<220>
   <223> Human IFN alpha-17 protein
<400> 18
   Cys Asp Leu Pro Gln Thr His Ser Leu Gly Asn Arg Arg Ala Leu Ile
   1 5 10 15
   Leu Leu Ala Gln Met Gly Arg Ile Ser Pro Phe Ser Cys Leu Lys Asp
   20 25 30
   Arg His Asp Phe Gly Leu Pro Gln Glu Glu Phe Asp Gly Asn Gln Phe
   35 40 45
   Gln Lys Thr Gln Ala Ile Ser Val Leu His Glu Met Ile Gln Gln Thr
   50 55 60
   Phe Asn Leu Phe Ser Thr Glu Asp Ser Ser Ala Ala Trp Glu Gln Ser
   65 70 75 80
   Leu Leu Glu Lys Phe Ser Thr Glu Leu Tyr Gln Gln Leu Asn Asn Leu
   85 90 95
   Glu Ala Cys Val Ile Gln Glu Val Gly Met Glu Glu Thr Pro Leu Met
   100 105 110
   Asn Glu Asp Ser Ile Leu Ala Val Arg Lys Tyr Phe Gln Arg Ile Thr
   115 120 125
   Leu Tyr Leu Thr Glu Lys Lys Tyr Ser Pro Cys Ala Trp Glu Val Val
   130 135 140
   Arg Ala Glu Ile Met Arg Ser Leu Ser Phe Ser Thr Asn Leu Gln Lys
   145 150 155 160
   Ile Leu Arg Arg Lys Asp
   165
<210> 19
   <211> 166
   <212> PRT
   <213> Homo sapiens
<220>
   <223> Human IFN alpha-21 protein
<400> 19
   Cys Asp Leu Pro Gln Thr His Ser Leu Gly Asn Arg Arg Ala Leu Ile
    1               5                  10                  15
   Leu Leu Ala Gln Met Gly Arg Ile Ser Pro Phe Ser Cys Leu Lys Asp
               20                  25                  30
   Arg His Asp Phe Gly Phe Pro Gln Glu Glu Phe Asp Gly Asn Gln Phe
           35                  40                  45
   Gln Lys Ala Gln Ala Ile Ser Val Leu His Glu Met Ile Gln Gln Thr
       50                  55                  60
   Phe Asn Leu Phe Ser Thr Lys Asp Ser Ser Ala Thr Trp Glu Gln Ser
   65                  70                  75                  80
   Leu Leu Glu Lys Phe Ser Thr Glu Leu Asn Gln Gln Leu Asn Asp Leu
                   85                  90                  95
   Glu Ala Cys Val Ile Gln Glu Val Gly Val Glu Glu Thr Pro Leu Met
               100                 105                 110
   Asn Val Asp Ser Ile Leu Ala Val Lys Lys Tyr Phe Gln Arg Ile Thr
           115                 120                 125
   Leu Tyr Leu Thr Glu Lys Lys Tyr Ser Pro Cys Ala Trp Glu Val Val
       130                 135                 140
   Arg Ala Glu Ile Met Arg Ser Phe Ser Leu Ser Lys Ile Phe Gln Glu
   145                 150                 155                 160
   Arg Leu Arg Arg Lys Glu
                   165
<210> 20
   <211> 172
   <212> PRT
   <213> Homo sapiens
<220>
   <223> Human IFN delta-1 protein
<400> 20
   Cys Asp Leu Ser Gln Asn His Val Leu Val Gly Arg Lys Asn Leu Arg
    1               5                  10                  15
   Leu Leu Asp Glu Met Arg Arg Leu Ser Pro His Phe Cys Leu Gln Asp
               20                  25                  30
   Arg Lys Asp Phe Ala Leu Pro Gln Glu Met Val Glu Gly Gly Gln Leu
           35                  40                  45
   Gln Glu Ala Gln Ala Ile Ser Val Leu His Glu Met Leu Gln Gln Ser
       50                  55                  60
   Phe Asn Leu Phe His Thr Glu His Ser Ser Ala Ala Trp Asp Thr Thr
    65                 70                  75                  80
   Leu Leu Glu Pro Cys Arg Thr Gly Leu His Gln Gln Leu Asp Asn Leu
                   85                  90                  95
   Asp Ala Cys Leu Gly Gln Val Met Gly Glu Glu Asp Ser Ala Leu Gly
               100                 105                 110
   Arg Thr Gly Pro Thr Leu Ala Leu Lys Arg Tyr Phe Gln Gly Ile His
           115                 120                 125
   Val Tyr Leu Lys Glu Lys Gly Tyr Ser Asp Cys Ala Trp Glu Thr Val
       130                 135                 140
   Arg Leu Glu Ile Met Arg Ser Phe Ser Ser Leu Ile Ser Leu Gln Glu
   145                 150                 155                 160
   Arg Leu Arg Met Met Asp Gly Asp Leu Ser Ser Pro
                   165                 170
<210> 21
   <211> 172
   <212> PRT
   <213> Homo sapiens
<220>
   <223> Human IFN omega-1 protein
<400> 21
   Cys Asp Leu Pro Gln Asn His Gly Leu Leu Ser Arg Asn Thr Leu Val
   1               5                   10                  15
   Leu Leu His Gln Met Arg Arg Ile Ser Pro Phe Leu Cys Leu Lys Asp
               20                  25                  30
   Arg Arg Asp Phe Arg Phe Pro Gln Glu Met Val Lys Gly Ser Gln Leu
           35                  40                  45
   Gln Lys Ala His Val Met Ser Val Leu His Glu Met Leu Gln Gln Ile
       50                  55                  60
   Phe Ser Leu Phe His Thr Glu Arg Ser Ser Ala Ala Trp Asn Met Thr
   65                  70                  75                  80
   Leu Leu Asp Gln Leu His Thr Gly Leu His Gln Gln Leu Gln His Leu
                   85                  90                  95
   Glu Thr Cys Leu Leu Gln Val Val Gly Glu Gly Glu Ser Ala Gly Ala
               100                 105                 110
   Ile Ser Ser Pro Ala Leu Thr Leu Arg Arg Tyr Phe Gln Gly Ile Arg
           115                 120                 125
   Val Tyr Leu Lys Glu Lys Lys Tyr Ser Asp Cys Ala Trp Glu Val Val
       130                 135                 140
   Arg Met Glu Ile Met Lys Ser Leu Phe Leu Ser Thr Asn Met Gln Glu
   145                 150                 155                 160
   Arg Leu Arg Ser Lys Asp Arg Asp Leu Gly Ser Ser
                   165                 170
<210> 22
   <211> 166
   <212> PRT
   <213> Mus musculus
<220>
   <223> Mouse IFN alpha-1 protein
<400> 22
   Cys Asp Leu Pro Gln Thr His Asn Leu Arg Asn Lys Arg Ala Leu Thr
   1               5                   10                  15
   Leu Leu Val Gln Met Arg Arg Leu Ser Pro Leu Ser Cys Leu Lys Asp
               20                  25                  30
   Arg Lys Asp Phe Gly Phe Pro Gln Glu Lys Val Asp Ala Gln Gln Ile
           35                  40                  45
   Lys Lys Ala Gln Ala Ile Pro Val Leu Ser Glu Leu Thr Gln Gln Ile
       50                  55                  60
   Leu Asn Ile Phe Thr Ser Lys Asp Ser Ser Ala Ala Trp Asn Ala Thr
   65                  70                  75                  80
   Leu Leu Asp Ser Phe Cys Asn Asp Leu His Gln Gln Leu Asn Asp Leu
                   85                  90                  95
   Gln Gly Cys Leu Met Gln Gln Val Gly Val Gln Glu Phe Pro Leu Thr
               100                 105                 110
   Gln Glu Asp Ala Leu Leu Ala Val Arg Lys Tyr Phe His Arg Ile Thr
           115                 120                 125
   Val Tyr Leu Arg Glu Lys Lys His Ser Pro Cys Ala Trp Glu Val Val
       130                 135                 140
   Arg Ala Glu Val Trp Arg Ala Leu Ser Ser Ser Ala Asn Val Leu Gly
   145                 150                 155                 160
   Arg Leu Arg Glu Glu Lys
                   165
<210> 23
   <211> 167
   <212> PRT
   <213> Mus musculus
<220>
   <223> Mouse IFN alpha-2 protein
<400> 23
   Cys Asp Leu Pro His Thr Tyr Asn Leu Arg Asn Lys Arg Ala Leu Lys
   1               5                   10                  15
   Val Leu Ala Gln Met Arg Arg Leu Pro Phe Leu Ser Cys Leu Lys Asp
               20                  25                  30
   Arg Gln Asp Phe Gly Phe Pro Leu Glu Lys Val Asp Asn Gln Gln Ile
           35                  40                  45
   Gln Lys Ala Gln Ala Ile Pro Val Leu Arg Asp Leu Thr Gln Gln Thr
        50                  55                  60
   Leu Asn Leu Phe Thr Ser Lys Ala Ser Ser Ala Ala Trp Asn Ala Thr
    65                  70                  75                  80
   Leu Leu Asp Ser Phe Cys Asn Asp Leu His Gln Gln Leu Asn Asp Leu
                    85                  90                  95
   Gln Thr Cys Leu Met Gln Gln Val Gly Val Gln Glu Pro Pro Leu Thr
               100                 105                 110
   Gln Glu Asp Ala Leu Leu Ala Val Arg Lys Tyr Phe His Arg Ile Thr
           115                 120                 125
   Val Tyr Leu Arg Glu Lys Lys His Ser Pro Cys Ala Trp Glu Val Val
       130                 135                 140
   Arg Ala Glu Val Trp Arg Ala Leu Ser Ser Ser Val Asn Leu Leu Pro
   145                 150                 155                 160
   Arg Leu Ser Glu Glu Lys Glu
                   165
<210> 24
   <211> 162
   <212> PRT
   <213> Mus musculus
<220>
   <223> Mouse IFN alpha-4 protein
<400> 24
   Cys Asp Leu Pro His Thr Tyr Asn Leu Gly Asn Lys Arg Ala Leu Thr
    1               5                  10                  15
   Val Leu Glu Glu Met Arg Arg Leu Pro Pro Leu Ser Cys Leu Lys Asp
               20                  25                  30
   Arg Lys Asp Phe Gly Phe Pro Leu Glu Lys Val Asp Asn Gln Gln Ile
           35                  40                  45
   Gln Lys Ala Gln Ala Ile Leu Val Leu Arg Asp Leu Thr Gln Gln Ile
       50                  55                  60
   Leu Asn Leu Phe Thr Ser Lys Asp Leu Ser Ala Thr Trp Asn Ala Thr
   65                  70                  75                  80
   Leu Leu Asp Ser Phe Cys Asn Asp Leu His Gln Gln Leu Asn Asp Leu
                   85                  90                  95
   Lys Ala Cys Val Met Gln Glu Pro Pro Leu Thr Gln Glu Asp Ser Leu
               100                 105                 110
   Leu Ala Val Arg Thr Tyr Phe His Arg Ile Thr Val Tyr Leu Arg Lys
           115                 120                 125
   Lys Lys His Ser Leu Cys Ala Trp Glu Val Ile Arg Ala Glu Val Trp
       130                 135                 140
   Arg Ala Leu Ser Ser Ser Thr Asn Leu Leu Ala Arg Leu Ser Glu Glu
   145                 150                 155                 160
   Lys Glu
<210> 25
   <211> 166
   <212> PRT
   <213> Mus musculus
<220>
   <223> Mouse IFN alpha-5 protein
<400> 25
   Cys Asp Leu Leu Gln Thr His Asn Leu Arg Asn Lys Arg Ala Leu Thr
    1               5                  10                  15
   Leu Leu Val Lys Met Arg Arg Leu Ser Pro Leu Ser Cys Leu Lys Asp
               20                  25                  30
   Arg Lys Asp Phe Gly Phe Pro Gln Glu Lys Val Gly Ala Gln Gln Ile
           35                  40                  45
   Gln Glu Ala Gln Ala Ile Pro Val Leu Ser Glu Leu Thr Gln Gln Val
       50                  55                  60
   Leu Asn Ile Phe Thr Ser Lys Asp Ser Ser Ala Ala Trp Asn Ala Thr
   65                  70                  75                  80
   Leu Leu Asp Ser Phe Cys Asn Glu Val His Gln Gln Leu Asn Asp Leu
                   85                  90                  95
   Lys Ala Cys Val Met Gln Gln Val Gly Val Gln Glu Ser Pro Leu Thr
               100                 105                 110
   Gln Glu Asp Ser Leu Leu Ala Val Arg Lys Tyr Phe His Arg Ile Thr
           115                 120                 125
   Val Tyr Leu Arg Glu Lys Lys His Ser Pro Cys Ala Trp Glu Val Val
       130                 135                 140
   Arg Ala Glu Val Trp Arg Ala Leu Ser Ser Ser Val Asn Leu Leu Ala
   145                 150                 155                 160
   Arg Leu Ser Lys Glu Glu
                   165
<210> 26
   <211> 166
   <212> PRT
   <213> Mus musculus
<220>
   <223> Mouse IFN alpha-6 protein
<400> 26
   Cys Asp Leu Pro Gln Thr His Asn Leu Arg Asn Lys Arg Ala Leu Thr
    1               5                  10                  15
   Leu Leu Val Lys Met Arg Arg Leu Ser Pro Leu Ser Cys Leu Lys Asp
               20                  25                  30
   Arg Lys Asp Phe Gly Phe Pro Gln Glu Lys Val Gly Ala Gln Gln Ile
           35                  40                  45
   Gln Glu Ala Gln Ala Ile Pro Val Leu Thr Glu Leu Thr Gln Gln Ile
       50                  55                  60
   Leu Thr Leu Phe Thr Ser Lys Asp Ser Ser Ala Ala Trp Asn Ala Thr
   65                  70                  75                  80
   Leu Leu Asp Ser Phe Cys Asn Asp Leu His Gln Leu Leu Asn Asp Leu
                   85                  90                  95
   Gln Gly Cys Leu Met Gln Gln Val Glu Ile Gln Ala Leu Pro Leu Thr
               100                 105                 110
   Gln Glu Asp Ser Leu Leu Ala Val Arg Thr Tyr Phe His Arg Ile Thr
           115                 120                 125
   Val Phe Leu Arg Glu Lys Lys His Ser Pro Cys Ala Trp Glu Val Val
       130                 135                 140
   Arg Ala Glu Val Trp Arg Ala Leu Ser Ser Ser Ala Lys Leu Leu Ala
   145                 150                 155                 160
   Arg Leu Asn Glu Asp Glu
                   165
<210> 27
   <211> 167
   <212> PRT
   <213> Mus musculus
<220>
   <223> Mouse IFN alpha-7 protein
<400> 27
   Cys Asp Leu Pro Gln Thr His Asn Leu Arg Asn Lys Arg Ala Leu Thr
   1 5 10 15
   Leu Leu Val Lys Met Arg Arg Leu Ser Pro Leu Ser Cys Leu Lys Asp
   20 25 30
   Arg Lys Asp Phe Gly Phe Pro Gln Ala Lys Val Asp Ala Gln Gln Ile
   35 40 45
   Gln Glu Ala Gln Ala Ile Pro Val Leu Ser Glu Leu Thr Gln Gln Ile
   50 55 60
   Leu Asn Ile Phe Thr Ser Lys Asp Ser Ser Ala Ala Trp Asn Ala Thr
   65 70 75 80
   Leu Leu Asp Ser Val Cys Asn Asp Leu His Gln Gln Leu Asn Asp Leu
   85 90 95
   Gln Gly Cys Leu Met Gln Glu Val Gly Val Gln Glu Leu Ser Leu Thr
   100 105 110
   Gln Glu Asp Ser Leu Leu Ala Val Arg Lys Tyr Phe His Arg Ile Thr
   115 120 125
   Val Phe Leu Arg Glu Lys Lys His Ser Pro Cys Ala Trp Glu Val Val
   130 135 140
   Arg Ala Glu Ile Trp Arg Ala Leu Ser Ser Ser Ala Asn Leu Leu Ala
   145 150 155 160
   Arg Leu Ser Glu Lys Lys Glu
   165
<210> 28
   <211> 166
   <212> PRT
   <213> Mus musculus
<220>
   <223> Mouse IFN alpha-8 protein
<400> 28
   Cys Asp Leu Pro Gln Thr His Asn Leu Arg Asn Lys Arg Ala Leu Thr
   1               5                   10                  15
   Leu Leu Val Lys Met Arg Arg Leu Ser Pro Leu Ser Cys Leu Lys Asp
               20                  25                  30
   Arg Lys Asp Phe Gly Phe Pro Gln Glu Lys Val Gly Ala Gln Gln Ile
           35                  40                  45
   Gln Glu Ala Gln Ala Ile Pro Val Leu Thr Glu Leu Thr Gln Gln Ile
       50                  55                  60
   Leu Ala Leu Phe Thr Ser Lys Asp Ser Ser Ala Ala Trp Asn Ala Thr
   65                  70                  75                  80
   Leu Leu Asp Ser Phe Cys Asn Asp Leu His Gln Leu Leu Asn Asp Leu
                   85                  90                  95
   Gln Gly Cys Leu Met Gln Gln Val Glu Ile Gln Ala Leu Pro Leu Thr
               100                 105                 110
   Gln Glu Asp Ser Leu Leu Ala Val Arg Thr Tyr Phe His Arg Ile Thr
           115                 120                 125
   Val Phe Leu Arg Glu Lys Lys His Ser Pro Cys Ala Trp Glu Val Val
       130                 135                 140
   Arg Ala Glu Val Trp Arg Ala Leu Ser Ser Ser Ala Lys Leu Leu Ala
   145                 150                 155                 160
   Arg Leu Asn Glu Asp Glu
                   165
<210> 29
   <211> 167
   <212> PRT
   <213> Mus musculus
<220>
   <223> Mouse IFN alpha-9 protein
<400> 29
   Cys Asp Leu Pro Gln Thr His Asn Leu Arg Asn Lys Lys Ile Leu Thr
    1               5                  10                  15
   Leu Leu Ala Gln Met Arg Arg Leu Ser Pro Leu Ser Cys Leu Lys Asp
               20                  25                  30
   Arg Lys Asp Phe Gly Phe Pro Gln Glu Lys Val Asp Ala Gln Gln Ile
           35                  40                  45
   Gln Glu Ala Gln Ala Ile Pro Val Leu Ser Glu Leu Thr Gln Gln Ile
       50                  55                  60
   Leu Thr Leu Phe Thr Ser Lys Asp Ser Ser Ala Ala Trp Asn Ala Thr
   65                  70                  75                  80
   Leu Leu Asp Ser Phe Cys Thr Gly Leu His Gln Leu Leu Asn Asp Leu
                   85                  90                  95
   Gln Gly Cys Leu Met Gln Leu Val Gly Met Lys Glu Leu Pro Leu Thr
               100                 105                 110
   Gln Glu Asp Ser Gln Leu Ala Met Lys Lys Tyr Phe His Arg Ile Thr
           115                 120                 125
   Val Tyr Leu Arg Glu Lys Lys His Ser Pro Cys Ala Trp Glu Val Val
       130                 135                 140
   Arg Ala Glu Val Trp Arg Ala Leu Ser Ser Ser Val Asn Leu Leu Ala
   145                 150                 155                 160
   Arg Leu Ser Glu Glu Lys Glu
                   165